# EUROPEAN PATENT APPLICATION

(11) **EP 4 417 231 A1**
(43) Date of publication of application: **21.08.2024**
(21) Application number: 24157302.1
(22) Date of filing: 13.02.2024
(51) Int. Cl.: A61M 5/32

(54) **DEVICE FOR PROVIDING CONTAINER CLOSURE INTEGRITY AND METHODS OF USE THEREOF**

(30) Priority: 14.02.2023 US 202363445314 P
(71) Applicant: Dali Medical Devices Ltd., 8122503 Yavne (IL)
(72) Inventor: RADAY, Lior, 7915200 Kibbutz Bror-Hail (IL); CARMEL, Ehoud, 5591618 Ganey Tikva (IL)
(74) Representative: Betten & Resch

(57) **Abstract**

A medical container with a needle situated within a volume enclosed in a contamination-barrier manner from the environment, comprising a syringe barrel with a piston slidably disposed therewithin; the syringe having a syringe outlet portion at a forward end thereof; the needle coupled with the syringe outlet portion; a needle cover being removably coupled to the syringe outlet portion and defining the volume; a barrier element coupled to the syringe outlet portion in a contamination-barrier providing manner, such that entrance of contaminants into the volume from the environment is prohibited when the barrier element is coupled to the syringe outlet portion.

## Description

### FIELD OF THE INVENTION

The present invention generally relates to a medical container with a needle attached thereto, and more specifically to providing container closure integrity for the needle and the contents of the medical container.

### BACKGROUND OF THE INVENTION

Various medical containers, such as syringes and cartridges, are known in the art. A needle can be fixedly mounted to a needle hub of the medical container or threadably attached thereto. The needle is usually covered by a needle cap and the connection between the needle hub and the needle cap is usually not airtight and thus the needle and the contents of the medical container may become contaminated.

A solution is required for this problem of contamination before usage of the syringe.

### SUMMARY OF THE INVENTION

The present invention seeks to provide a device for providing a container closure integrity and methods of use thereof.

There is thus provided in accordance with an embodiment of the present invention or a combination of embodiments thereof a medical container with a needle situated within a volume enclosed in a contamination-barrier manner from the environment, comprising: a syringe barrel with a piston slidably disposed therewithin; the syringe having a syringe outlet portion at a forward end thereof; the needle coupled with the syringe outlet portion; a needle cover being removably coupled to the syringe outlet portion and defining the volume; a barrier element coupled to the syringe outlet portion in a contamination-barrier providing manner, such that entrance of contaminants into the volume from the environment is prohibited when the barrier element is coupled to the syringe outlet portion.

Preferably, a luer portion is formed at the syringe outlet portion. Further preferably, a needle hub is coupled or integrally attached with the luer portion and the needle being fixedly attached to the needle hub. Still further preferably, the barrier element is coupled to at least one of the needle hub or the syringe outlet portion. Yet further preferably, the barrier element is also coupled to the needle cover.

In accordance with an embodiment of the present invention, the barrier element is disposed between an outer surface of the needle hub and an inner surface of the needle cover.

Preferably, the barrier element is coupled both to the luer portion and to the needle cover and is formed as a resilient sleeve. Further preferably, the barrier element is removably attached to the luer portion. Alternatively, the barrier element is fixedly attached to the luer portion.

In accordance with an embodiment of the present invention, the barrier element is attached to both an outer surface of the needle hub and an outer surface of the needle cover. Preferably, the medical container further comprises an o-ring disposed between the outer surface of the needle hub and the barrier element. Further preferably, the barrier element is attached to both an outer surface of the luer portion and an outer surface of the needle cover. Still further preferably, the barrier element is fixedly attached to or integrally molded with the needle hub. Yet further preferably, the barrier element is mounted over the needle cover and engages an outer portion of the luer portion.

In accordance with an embodiment of the present invention, the medical container further comprises a vent element selectably operatively engaged with the barrier element to allow passage of fluid into an inner volume of the barrier element in a first operative orientation and prevent passage of fluid into the inner volume of the barrier element in a second operative orientation. Preferably, the vent element is operable by a user. Alternatively, the vent element is operable upon exertion of pressure change.

In accordance with an embodiment of the present invention, and whereas the vent element is fixedly attached to a package of the medical container. Preferably, the medical container further comprises a reinforcing sleeve received by the barrier element.

In accordance with an embodiment of the present invention, a needle sub-assembly situated within a volume enclosed in a contamination-barrier manner from the environment, comprising a needle hub configured to be coupled or integrally attached with a luer portion of a medical container, a needle being fixedly attached to the needle hub; a needle cover being removably coupled to the needle hub; a barrier element, which engages at least one of the needle hub, the luer portion or the needle cover in a contamination-barrier providing manner.

In accordance with an embodiment of the present invention, a medical container with a needle situated within a volume enclosed in a contamination-barrier manner from the environment, comprising a syringe barrel with a piston slidably disposed therewithin; the syringe having a luer portion at a forward end thereof; a needle hub coupled or integrally attached with the luer portion, the needle being fixedly attached to the needle hub; a needle cover being removably coupled to at least one of the luer portion or the needle hub in a contamination-barrier providing manner.

Preferably, the medical container further comprising a barrier element, which engages at least one of the needle hub, the needle cover or the luer portion in a contamination-barrier providing manner. Further preferably, the needle cover is coupled both to an outer surface of the luer portion and to the needle hub. Still further preferably, an outer surface of the needle cover is coupled to an inner surface of an external luer portion, forming part of the luer portion.

### BRIEF DESCRIPTION OF THE DRAWINGS

The present invention will be understood and appreciated more fully from the following detailed description, taken in conjunction with the drawings in which:
Figs. 1A and 1B are respectively simplified planar side view and partial sectional view taken along lines B - B in Fig. 1A of a medical container with a covered needle attached thereto, constructed and operative in accordance with a first embodiment of the present invention;
Figs. 2A and 2B are respectively simplified planar side view and partial sectional view taken along lines B - B in Fig. 2A of a medical container with a covered needle attached thereto, constructed and operative in accordance with a second embodiment of the present invention;
Figs. 3A and 3B are respectively simplified planar side view and partial sectional view taken along lines B - B in Fig. 3A of a medical container with a covered needle attached thereto, constructed and operative in accordance with a third embodiment of the present invention;
Figs. 4A and 4B are respectively simplified planar side view and partial sectional view taken along lines B - B in Fig. 4A of a medical container with a covered needle attached thereto, constructed and operative in accordance with a fourth embodiment of the present invention;
Figs. 5A and 5B are respectively simplified planar side view and partial sectional view taken along lines B - B in Fig. 5A of a medical container with a covered needle attached thereto, constructed and operative in accordance with a fifth embodiment of the present invention;
Figs. 6A and 6B are respectively simplified planar side view and partial sectional view taken along lines B - B in Fig. 6A of a medical container with a covered needle attached thereto, constructed and operative in accordance with a sixth embodiment of the present invention;
Figs. 7A and 7B are respectively simplified planar side view and partial sectional view taken along lines B - B in Fig. 7A of a medical container with a covered needle attached thereto, constructed and operative in accordance with a seventh embodiment of the present invention;
Figs. 8A and 8B are respectively simplified planar side view and partial sectional view taken along lines B - B in Fig. 8A of a medical container with a covered needle attached thereto, constructed and operative in accordance with an eighth embodiment of the present invention;
Figs. 9A and 9B are respectively simplified planar side view and partial sectional view taken along lines B - B in Fig. 9A of a medical container with a covered needle attached thereto, constructed and operative in accordance with a ninth embodiment of the present invention;
Figs. 10A and 10B are respectively simplified planar side view and partial sectional view taken along lines B - B in Fig. 10A of a medical container with a covered needle attached thereto, constructed and operative in accordance with a tenth embodiment of the present invention;
Figs. 11A, 11B and 11C are respectively simplified planar side view, a partial sectional view taken along lines B - B in Fig. 11A of a medical container with a covered needle attached thereto showing the device in a storage operative orientation and a partial sectional view taken along lines B - B in Fig. 11A of a medical container with a covered needle attached thereto showing the device in a use operative orientation, constructed and operative in accordance with an eleventh embodiment of the present invention;
Figs. 12A, 12B and 12C are respectively simplified planar side view, a partial sectional view taken along lines B - B in Fig. 12A of a medical container with a covered needle attached thereto showing the device in a storage operative orientation and a partial sectional view taken along lines B - B in Fig. 12A of a medical container with a covered needle attached thereto showing the device in use operative orientation, constructed and operative in accordance with a twelfth embodiment of the present invention;
Figs. 13A and 13B are respectively simplified planar side view and a partial sectional view taken along lines B - B in Fig. 13A of a medical container with a covered needle attached thereto showing the device in a storage operative orientation, constructed and operative in accordance with a thirteenth embodiment of the present invention;
Figs. 13C and 13D are respectively simplified planar side view and a partial sectional view taken along lines D - D in Fig. 13C of a medical container with a covered needle attached thereto showing the device in use operative orientation, constructed and operative in accordance with the thirteenth embodiment of the present invention;
Figs. 14A and 14B are respectively simplified planar side view and partial sectional view taken along lines B - B in Fig. 14A of a medical container with a covered needle attached thereto, constructed and operative in accordance with a fourteenth embodiment of the present invention.

### DESCRIPTION OF EMBODIMENTS

The principles, uses and implementations of the teachings herein may be better understood with reference to the accompanying description and figures. Upon perusal of the description and figures present herein, one skilled in the art is able to implement the invention without undue effort or experimentation.

Before explaining at least one embodiment of the invention in detail, it is to be understood that the invention is not limited in its applications to the details of construction and the arrangement of the components and/or methods set forth in the following description and/or illustrated in the drawings and/or the Examples. The invention can be implemented with other embodiments and can be practiced or carried out in various ways. It is also understood that the phraseology and terminology employed herein is for descriptive purpose and should not be regarded as limiting.

Some embodiments of the invention are described herein with reference to the accompanying figures. The description, together with the figures, makes apparent to a person having ordinary skill in the art how some embodiments of the invention may be practiced. The figures are for the purpose of illustrative discussion and no attempt is made to show structural details of an embodiment in more detail than is necessary for a fundamental understanding of the invention. For the sake of clarity, some objects depicted in the figures are not to scale.

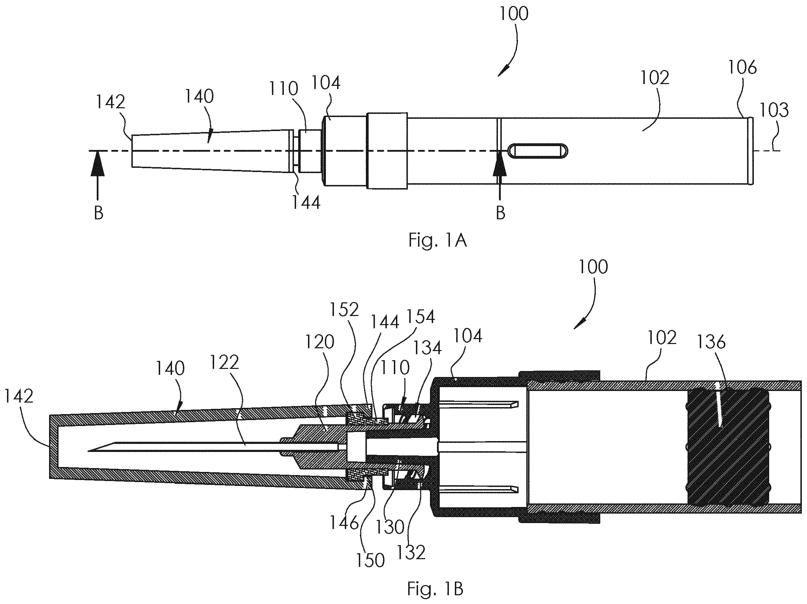

Reference is now made to Figs. 1A and 1B, which are respectively simplified planar side view and partial sectional view taken along lines B - B in Fig. 1A of a medical container with a covered needle attached thereto, constructed and operative in accordance with a first embodiment of the present invention.

As seen in Figs. 1A & 1B, a medical container 100 preferably includes a syringe barrel 102 arranged along a longitudinal axis 103 and having a forward end 104 and a rearward end 106. A luer portion 110 is formed at the forward end 104 of the syringe barrel 102 and extends longitudinally forwardly therefrom. A needle hub 120 is attached to the luer portion 110 and a needle 122 is fixedly attached to the needle hub 120.

It is noted that the medical container 100 may be any container known in the art, such as a pre-filled syringe, in which case the needle 122 is fixedly attached to the luer portion 110 of the container 100 and thus the luer portion 110 serves as the needle hub 120. Alternatively, the medical container 100 may be a cartridge with a luer portion 110 whereas the needle hub 120 with the needle 122 is attached to the luer portion 110.

It is appreciated that medical container 100 can be any type of conventional container, such as a cartridge commercially available from Schott Pharmaceutical Systems, Mainz, Germany or Vetter Pharma International USA Inc., IL, USA or Nuova Ompi S.r.l., Padua, Italy or may be any other suitable syringe or cartridge.

The luer portion 110 in accordance with the first embodiment of the present invention includes a luer lock, which has an inner luer portion 130 and an external luer portion 132, which generally surrounds the inner luer portion 130 and has an internal threading 134. In accordance with this embodiment of the present invention, the needle hub 120 is threadably attached to the luer portion 110 in a sealing manner, thereby providing sealing relationship between the needle hub 120 and the inner luer portion 130. It is noted that the luer portion 110 may alternatively include a luer slip, where no external luer portion, such as portion 132, is provided.

It is noted that alternatively another type of connector may be provided at the forward end 104 of the syringe barrel 102, instead of the luer portion 110, such as a tube connector or any other mechanical connector.

It is noted that further alternatively, the luer portion and the needle hub may be obviated and the needle can be fixedly attached directly to the forward end of the medical container 100, which serves as the syringe outlet portion. The needle 122 may be fixed to the syringe outlet portion using an adhesive for example or any other kind of permanent attachment to the syringe outlet portion.

The luer portion 110 may be an integral portion of the medical container 100 or may be attached to the front end 104 thereof.

It is also seen in Fig. 1B that at least one piston 136 is contained within the medical container 100 and adapted for slidable axial displacement relative to the syringe barrel 102. It is appreciated that the syringe barrel 102 may contain a single substance contained by a single piston or several substances, each one contained between a pair of pistons. The at least one piston 136 may also define a fluid chamber to be filled following the process of reconstitution or mixing of several substances, as particularly seen in the embodiment shown in Figs. 1A & 1B.

It is a particular feature of an embodiment of the present invention that a needle cover 140 is removably coupled to the needle hub 120 in a contamination-barrier providing manner. The needle cover 140 has a closed forward end 142 and an open rearward end 144, preferably defining a forwardly facing flange 146. Specifically, it is seen in Fig. 1B that the needle cover 140 has a barrier element 150, that is preferably fixedly attached to the rearward end 144 thereof. The barrier element 150 is preferably made of a resilient material, such as silicon or TPE and may be fixedly retained within the needle cover 140 by means of a groove formed forwardly of the forwardly facing flange 146. Alternatively, the barrier element 150 may be integrally molded with the needle cover 140. Further alternatively, the barrier element 150 may be integrally molded with the needle hub 120.

It is seen in Fig. 1B that in accordance with the first embodiment of the present invention the barrier element 150 includes a retaining portion 152 at a forward end thereof, which is adapted for attachment with the needle cover 140 and a contamination-barrier providing portion 154, which is a generally longitudinal cylindrical portion, which is adapted to be mounted over the needle hub 120, such that the inner circumference of the contamination-barrier providing portion 154 engages the outer circumference of the needle hub 120, thereby preventing from any contaminants to enter between the needle hub 120 and the needle cover 140 and thereby provides for sterile environment within the inner volume of the needle cover 140 as long as it is coupled to the needle hub 120. The container closure integrity is thereby maintained as long as the needle cover 140 is coupled to the needle hub 120.

It is particularly noted that according to an embodiment in which the barrier element 150 is a separate element, it is mounted both onto the needle hub 120 and into the needle cover 140 in a pressure-fit manner. According to an embodiment in which the barrier element 150 is molded together with the needle cover 140, it is mounted onto the needle hub 120 in a pressure-fit manner. Further, according to an embodiment in which the barrier element 150 is molded together with the needle hub120, it is mounted into the needle cover 140 in a pressure-fit manner.

These pressure-fit engagements provide for contamination-barrier for the inner volume of the needle cover 140, the inner volume of the needle 122, the inner volume of the inner luer portion 130 and the inner volume of the syringe barrel 102 defined by the piston 136, all of these volumes are configured to fluidly communicate with each other.

Contamination-barrier is provided for prohibiting entrance of debris, harmful bacteria, viruses, other microorganisms, or any foreign particulates from the environment.

It is noted that the needle cover 140 with the barrier element 150 may be coupled onto the needle hub 120 either before or after the coupling of the needle hub 120 to the luer portion 110.

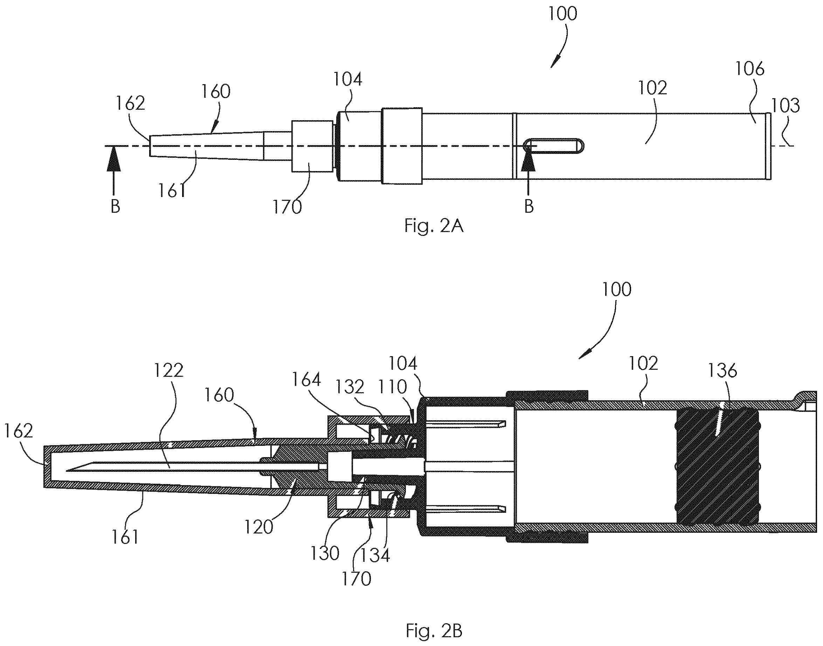

Reference is now made to Figs. 2A and 2B, which are respectively simplified planar side view and partial sectional view taken along lines B - B in Fig. 2A of a medical container with a covered needle attached thereto, constructed and operative in accordance with a second embodiment of the present invention.

As seen in Figs. 2A & 2B, a medical container 100 preferably includes a syringe barrel 102 arranged along a longitudinal axis 103 and having a forward end 104 and a rearward end 106. A luer portion 110 is formed at the forward end 104 of the syringe barrel 102 and extends longitudinally forwardly therefrom. A needle hub 120 is attached to the luer portion 110 and a needle 122 is fixedly attached to the needle hub 120.

It is noted that the medical container 100 may be any container known in the art, such as a pre-filled syringe, in which case the needle 122 is fixedly attached to the luer portion 110 of the container 100 and thus the luer portion 110 serves as the needle hub 120. Alternatively, the medical container 100 may be a cartridge with a luer portion 110 whereas the needle hub 120 with the needle 122 is attached to the luer portion 110.

It is appreciated that medical container 100 can be any type of conventional container, such as a cartridge commercially available from Schott Pharmaceutical Systems, Mainz, Germany or Vetter Pharma International USA Inc., IL, USA or Nuova Ompi S.r.l., Padua, Italy or may be any other suitable syringe or cartridge.

The luer portion 110 in accordance with the second embodiment of the present invention includes a luer lock, which has an inner luer portion 130 and an external luer portion 132, which generally surrounds the inner luer portion 130 and has an internal threading 134. In accordance with this embodiment of the present invention, the needle hub 120 is threadably attached to the luer portion 110 in a sealing manner, thereby providing sealing relationship between the needle hub 120 and the inner luer portion 130. It is noted that the luer portion 110 may alternatively include a luer slip, where no external luer portion, such as portion 132, is provided.

It is noted that alternatively another type of connector may be provided at the forward end 104 of the syringe barrel 102, instead of the luer portion 110, such as a tube connector or any other mechanical connector.

It is noted that further alternatively, the luer portion and the needle hub may be obviated and the needle can be fixedly attached directly to the forward end of the medical container 100, which serves as the syringe outlet portion. The needle 122 may be fixed to the syringe outlet portion using an adhesive for example or any other kind of permanent attachment to the syringe outlet portion.

The luer portion 110 may be an integral portion of the medical container 100 or may be attached to the front end 104 thereof.

It is also seen in Fig. 2B that at least one piston 136 is contained within the medical container 100 and adapted for slidable axial displacement relative to the syringe barrel 102. It is appreciated that the syringe barrel 102 may contain a single substance contained by a single piston or several substances, each one contained between a pair of pistons. The at least one piston 136 may also define a fluid chamber to be filled following the process of reconstitution or mixing of several substances, as particularly seen in the embodiment shown in Figs. 2A & 2B.

It is a particular feature of an embodiment of the present invention that a needle cover 160 is removably coupled to the luer portion 110 in a contamination-barrier providing manner. It is specifically seen in Fig. 2B that the needle cover 160 has a generally conical main sleeve 161, having a closed forward end 162 and an open rearward end 164. A barrier sleeve 170 is formed around the rearward portion of the main sleeve 161 of the needle cover 160. The barrier sleeve170 is generally arranged concentrically with the main sleeve 161 and extends rearwardly from a location adjacent the rearward end 164 of the main sleeve 161.

It is seen in Fig. 2B that the needle cover 160 in accordance with the second embodiment of the present invention is mounted over both the needle hub 120 and the luer portion 110, such that the inner circumference of the rearward portion of the main sleeve 161 engages the outer circumference of the needle hub 120 or alternatively is slightly radially spaced between the outer circumference of the needle hub 120 and the inner circumference of the external luer portion 132. The barrier sleeve170 engages the external luer portion 132 of the luer portion 110 in a pressure-fit contamination-barrier providing manner. Entrance of contaminants between the needle cover 160 and the external luer portion 132 of the luer portion 110 is prevented due to the pressure-fit engagement between the barrier sleeve170 and the external luer portion 132 and thereby sterile environment is provided within the inner volume of the needle cover 160 as long as it is coupled to the luer portion 110. The container closure integrity is thereby maintained as long as the needle cover 160 is coupled to the luer portion 110.

These pressure-fit engagements provide for contamination-barrier for the inner volume of the needle cover 160, the inner volume of the needle 122, the inner volume of sleeve 170, the inner volume of the luer portion 110 and the inner volume of the syringe barrel 102 defined by the piston 136, all of these volumes are configured to fluidly communicate with each other.

Contamination-barrier is provided for prohibiting entrance of debris, harmful bacteria, viruses, other microorganisms, or any foreign particulates from the environment.

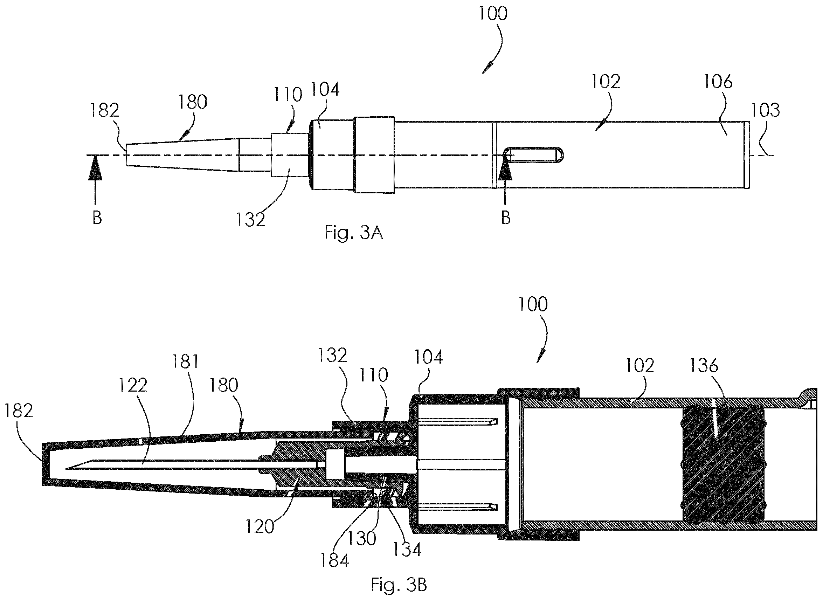

Reference is now made to Figs. 3A and 3B, which are respectively simplified planar side view and partial sectional view taken along lines B - B in Fig. 3A of a medical container with a covered needle attached thereto, constructed and operative in accordance with a third embodiment of the present invention.

As seen in Figs. 3A & 3B, a medical container 100 preferably includes a syringe barrel 102 arranged along a longitudinal axis 103 and having a forward end 104 and a rearward end 106. A luer portion 110 is formed at the forward end 104 of the syringe barrel 102 and extends longitudinally forwardly therefrom. A needle hub 120 is attached to the luer portion 110 and a needle 122 is fixedly attached to the needle hub 120.

It is noted that the medical container 100 may be any container known in the art, such as a pre-filled syringe, in which case the needle 122 is fixedly attached to the luer portion 110 of the container 100 and thus the luer portion 110 serves as the needle hub 120. Alternatively, the medical container 100 may be a cartridge with a luer portion 110 whereas the needle hub 120 with the needle 122 is attached to the luer portion 110.

It is appreciated that medical container 100 can be any type of conventional container, such as a cartridge commercially available from Schott Pharmaceutical Systems, Mainz, Germany or Vetter Pharma International USA Inc., IL, USA or Nuova Ompi S.r.l., Padua, Italy or may be any other suitable syringe or cartridge.

The luer portion 110 in accordance with the third embodiment of the present invention includes a luer lock, which has an inner luer portion 130 and an external luer portion 132, which generally surrounds the inner luer portion 130 and has an internal threading 134. In accordance with this embodiment of the present invention, the needle hub 120 is threadably attached to the luer portion 110 in a sealing manner, thereby providing sealing relationship between the needle hub 120 and the inner luer portion 130. It is noted that the luer portion 110 may alternatively include a luer slip, where no external luer portion, such as portion 132, is provided.

It is noted that alternatively another type of connector may be provided at the forward end 104 of the syringe barrel 102, instead of the luer portion 110, such as a tube connector or any other mechanical connector.

It is noted that further alternatively, the luer portion and the needle hub may be obviated and the needle can be fixedly attached directly to the forward end of the medical container 100, which serves as the syringe outlet portion. The needle 122 may be fixed to the syringe outlet portion using an adhesive for example or any other kind of permanent attachment to the syringe outlet portion.

The luer portion 110 may be an integral portion of the medical container 100 or may be attached to the front end 104 thereof.

It is also seen in Fig. 3B that at least one piston 136 is contained within the medical container 100 and adapted for slidable axial displacement relative to the syringe barrel 102. It is appreciated that the syringe barrel 102 may contain a single substance contained by a single piston or several substances, each one contained between a pair of pistons. The at least one piston 136 may also define a fluid chamber to be filled following the process of reconstitution or mixing of several substances, as particularly seen in the embodiment shown in Figs. 3A & 3B.

It is a particular feature of an embodiment of the present invention that a needle cover 180 is removably coupled to the luer portion 110 in a contamination-barrier providing manner. It is specifically seen in Fig. 3B that the needle cover 180 has a generally conical main sleeve 181, having a closed forward end 182 and an open rearward end 184.

It is seen in Fig. 3B that the needle cover 180 in accordance with the third embodiment of the present invention is mounted over the needle hub 120 and is partially inserted between the needle hub 120 and the external luer portion 132 of the luer portion 110. It is particularly seen in Fig. 3B that the rearward portion of the main sleeve 181 is inserted between the needle hub 120 and the external luer portion 132. The inner circumference of the rearward portion disposed adjacent the rearward end 184 of the main sleeve 181 engages the outer circumference of the needle hub 120 or alternatively is slightly spaced from the outer circumference of the needle hub 120 and the outer circumference of the rearward portion of the main sleeve 181 engages the internal circumference of the external luer portion 132 of the luer portion 110 in a pressure-fit contamination-barrier providing manner. Entrance of contaminants between the needle cover 180 and the external luer portion 132 of the luer portion 110 is prevented due to the pressure-fit engagement between the main sleeve 181 and the external luer portion 132 and thereby sterile environment is provided within the inner volume of the needle cover 180 as long as it is coupled to the luer portion 110. The container closure integrity is thereby maintained as long as the needle cover 180 is coupled to the luer portion 110.

These pressure-fit engagements provide for contamination-barrier for the inner volume of the needle cover 180, the inner volume of the needle 122, the inner volume of sleeve 170, the inner volume of the luer portion 110 and the inner volume of the syringe barrel 102 defined by the piston 136, all of these volumes are configured to fluidly communicate with each other.

Contamination-barrier is provided for prohibiting entrance of debris, harmful bacteria, viruses, other microorganisms, or any foreign particulates from the environment.

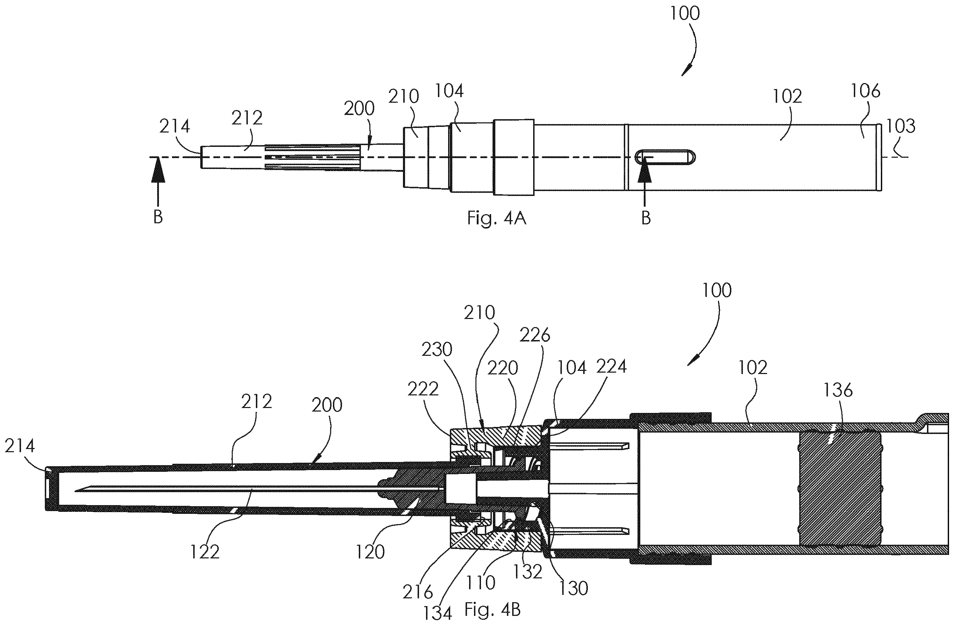

Reference is now made to Figs. 4A and 4B, which are respectively simplified planar side view and partial sectional view taken along lines B - B in Fig. 4A of a medical container with a covered needle attached thereto, constructed and operative in accordance with a fourth embodiment of the present invention.

As seen in Figs. 4A & 4B, a medical container 100 preferably includes a syringe barrel 102 arranged along a longitudinal axis 103 and having a forward end 104 and a rearward end 106. A luer portion 110 is formed at the forward end 104 of the syringe barrel 102 and extends longitudinally forwardly therefrom. A needle hub 120 is attached to the luer portion 110 and a needle 122 is fixedly attached to the needle hub 120.

It is noted that the medical container 100 may be any container known in the art, such as a pre-filled syringe, in which case the needle 122 is fixedly attached to the luer portion 110 of the container 100 and thus the luer portion 110 serves as the needle hub 120. Alternatively, the medical container 100 may be a cartridge with a luer portion 110 whereas the needle hub 120 with the needle 122 is attached to the luer portion 110.

It is appreciated that medical container 100 can be any type of conventional container, such as a cartridge commercially available from Schott Pharmaceutical Systems, Mainz, Germany or Vetter Pharma International USA Inc., IL, USA or Nuova Ompi S.r.l., Padua, Italy or may be any other suitable syringe or cartridge.

The luer portion 110 in accordance with the fourth embodiment of the present invention includes a luer lock, which has an inner luer portion 130 and an external luer portion 132, which generally surrounds the inner luer portion 130 and has an internal threading 134. In accordance with this embodiment of the present invention, the needle hub 120 is threadably attached to the luer portion 110 in a sealing manner, thereby providing sealing relationship between the needle hub 120 and the inner luer portion 130. It is noted that the luer portion 110 may alternatively include a luer slip, where no external luer portion, such as portion 132, is provided.

It is noted that alternatively another type of connector may be provided at the forward end 104 of the syringe barrel 102, instead of the luer portion 110, such as a tube connector or any other mechanical connector.

It is noted that further alternatively, the luer portion and the needle hub may be obviated and the needle can be fixedly attached directly to the forward end of the medical container 100, which serves as the syringe outlet portion. The needle 122 may be fixed to the syringe outlet portion using an adhesive for example or any other kind of permanent attachment to the syringe outlet portion.

The luer portion 110 may be an integral portion of the medical container 100 or may be attached to the front end 104 thereof.

It is also seen in Fig. 4B that at least one piston 136 is contained within the medical container 100 and adapted for slidable axial displacement relative to the syringe barrel 102. It is appreciated that the syringe barrel 102 may contain a single substance contained by a single piston or several substances, each one contained between a pair of pistons. The at least one piston 136 may also define a fluid chamber to be filled following the process of reconstitution or mixing of several substances, as particularly seen in the embodiment shown in Figs. 4A & 4B.

It is a particular feature of an embodiment of the present invention that a needle cover 200 is removably coupled to the needle hub 120 and a barrier sleeve 210 is mounted over the luer portion 110 and the rearward end of the needle cover 200 in a contamination-barrier providing manner.

It is noted that in accordance with an embodiment of the present invention, the needle sub-assembly comprising the needle hub 120 with the needle 122 covered by the needle cover 200 can be any type of conventional needle sub-assembly, such as the needle sub-assembly commercially available from Medicpro, Chungcheongnam-do, Korea or Medi-plus, New Jersey, United States or may be any other suitable needle sub-assembly.

It is specifically seen in Fig. 4B that the needle cover 200 has a generally conical main sleeve 212, having a closed forward end 214 and an open rearward end 216. It is also seen in Fig. 4B that the barrier sleeve 210 has a hollow cylindrical portion 220, a forward end 222 and a rearward end 224. The barrier sleeve 210 defines an inner surface 226. A protrusion 230 extends radially inwardly from the inner surface 226 of the barrier sleeve, and is preferably formed at a location adjacent the forward end 222 of the barrier sleeve 210. Alternatively, the barrier sleeve 210 may have a cylindrical inner circumference.

It is noted that the barrier sleeve 210 is preferably made of a resilient material, such as silicon or TPE and it is configured to be attached to the luer portion 110 after connection of the needle hub 120 to the luer portion 110.

It is seen in Fig. 4B that the needle cover 200 in accordance with the fourth embodiment of the present invention is mounted over the needle hub 120, such that the inner circumference of the rearward portion of the needle cover 200 engages the outer circumference of the needle hub 120. It is further particularly seen in Fig. 4B that the barrier sleeve 210 is mounted over a portion of the external luer portion 132 and the rearward portion of the needle cover 200, such that the inner surface 226 of the barrier sleeve 210 engages the outer circumference of the external luer portion 132 in a pressure-fit contamination-barrier providing manner. Further, the protrusion 230 of the barrier sleeve engages the rearward portion of the main sleeve 212 in a pressure-fit manner. These two pressure-fit engagements provide for contamination-barrier for the inner volume of the needle cover 200, the inner volume of the needle 122, the inner volume of the barrier sleeve 210, the inner volume of the luer portion 110 and the inner volume of the syringe barrel 102 defined by the piston 136. The rearward end 224 of the barrier sleeve 210 is preferably disposed adjacent the forward end 104 of the syringe barrel 102 and the forward end 222 of the barrier sleeve 210 is preferably disposed adjacent the rearward end 216 of the main sleeve 212 of the needle cover 200.

Contamination-barrier is provided for prohibiting entrance of debris, harmful bacteria, viruses, other microorganisms, or any foreign particulates from the environment.

Entrance of contaminants between the needle cover 200 and the barrier sleeve 210 is prevented due to the engagement between the protrusion 230 of the barrier sleeve 210 and the rearward portion of the needle cover 200 and thereby sterile environment is provided within the inner volume of the needle cover 200 as long as the barrier sleeve 210 is coupled to the needle cover 200. The container closure integrity is thereby maintained as long as the barrier sleeve 210 is coupled to the needle cover 200. Upon removal of the needle cover 200, the barrier sleeve 210 remains mounted onto the external luer portion 132 of the luer portion 110.

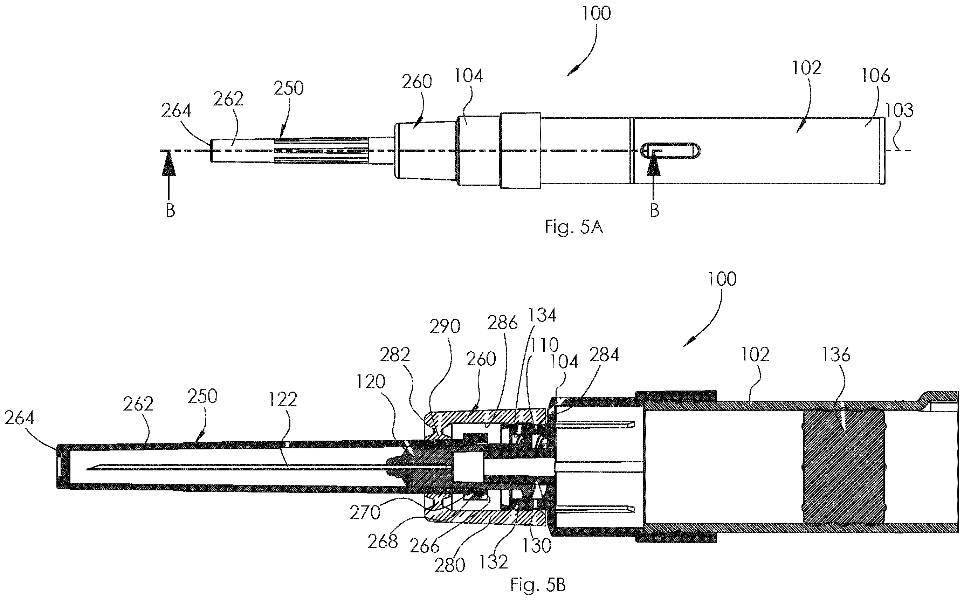

Reference is now made to Figs. 5A and 5B, which are respectively simplified planar side view and partial sectional view taken along lines B - B in Fig. 5A of a medical container with a covered needle attached thereto, constructed and operative in accordance with a fifth embodiment of the present invention.

As seen in Figs. 5A & 5B, a medical container 100 preferably includes a syringe barrel 102 arranged along a longitudinal axis 103 and having a forward end 104 and a rearward end 106. A luer portion 110 is formed at the forward end 104 of the syringe barrel 102 and extends longitudinally forwardly therefrom. A needle hub 120 is attached to the luer portion 110 and a needle 122 is fixedly attached to the needle hub 120.

It is noted that the medical container 100 may be any container known in the art, such as a pre-filled syringe, in which case the needle 122 is fixedly attached to the luer portion 110 of the container 100 and thus the luer portion 110 serves as the needle hub 120. Alternatively, the medical container 100 may be a cartridge with a luer portion 110 whereas the needle hub 120 with the needle 122 is attached to the luer portion 110.

It is appreciated that medical container 100 can be any type of conventional container, such as a cartridge commercially available from Schott Pharmaceutical Systems, Mainz, Germany or Vetter Pharma International USA Inc., IL, USA or Nuova Ompi S.r.l., Padua, Italy or may be any other suitable syringe or cartridge.

The luer portion 110 in accordance with the fifth embodiment of the present invention includes a luer lock, which has an inner luer portion 130 and an external luer portion 132, which generally surrounds the inner luer portion 130 and has an internal threading 134. In accordance with this embodiment of the present invention, the needle hub 120 is threadably attached to the luer portion 110 in a sealing manner, thereby providing sealing relationship between the needle hub 120 and the inner luer portion 130. It is noted that the luer portion 110 may alternatively include a luer slip, where no external luer portion, such as portion 132, is provided.

It is noted that alternatively another type of connector may be provided at the forward end 104 of the syringe barrel 102, instead of the luer portion 110, such as a tube connector or any other mechanical connector.

It is noted that further alternatively, the luer portion and the needle hub may be obviated and the needle can be fixedly attached directly to the forward end of the medical container 100, which serves as the syringe outlet portion. The needle 122 may be fixed to the syringe outlet portion using an adhesive for example or any other kind of permanent attachment to the syringe outlet portion.

The luer portion 110 may be an integral portion of the medical container 100 or may be attached to the front end 104 thereof.

It is also seen in Fig. 5B that at least one piston 136 is contained within the medical container 100 and adapted for slidable axial displacement relative to the syringe barrel 102. It is appreciated that the syringe barrel 102 may contain a single substance contained by a single piston or several substances, each one contained between a pair of pistons. The at least one piston 136 may also define a fluid chamber to be filled following the process of reconstitution or mixing of several substances, as particularly seen in the embodiment shown in Figs. 5A & 5B.

It is a particular feature of an embodiment of the present invention that a needle cover 250 is removably coupled to the needle hub 120 and a barrier sleeve 260 is mounted over the luer portion 110 and the rearward end of the needle cover 250 in a contamination-barrier providing manner.

It is noted that in accordance with an embodiment of the present invention, the needle sub-assembly comprising the needle hub 120 with the needle 122 covered by the needle cover 250 can be any type of conventional needle sub-assembly, such as the needle sub-assembly commercially available from Medicpro, Chungcheongnam-do, Korea or Medi-plus, New Jersey, United States or may be any other suitable needle sub-assembly.

It is specifically seen in Fig. 5B that the needle cover 250 has a generally conical main sleeve 262, having a closed forward end 264 and an open rearward end 266. An enlarged generally annular portion 268 is formed at the rearward end 266 of the needle cover 250 and forms a forwardly facing shoulder 270.

It is also seen in Fig. 5B that the barrier sleeve 260 has a hollow cylindrical portion 280, a forward end 282 and a rearward end 284. The barrier sleeve 260 defines an inner surface 286. A protrusion 290 extends radially inwardly from the inner surface 286 of the barrier sleeve 260, and is preferably formed at a location adjacent the forward end 282 of the barrier sleeve 260. Alternatively, the barrier sleeve 260 may have a cylindrical inner circumference.

It is noted that the barrier sleeve 260 is preferably made of a resilient material, such as silicon or TPE and it is configured to be attached to the luer portion 110 after connection of the needle hub 120 to the luer portion 110.

It is seen in Fig. 5B that the needle cover 250 in accordance with the fifth embodiment of the present invention is mounted over the needle hub 120, such that the inner circumference of the rearward portion of the needle cover 250 engages the outer circumference of the needle hub 120. It is further particularly seen in Fig. 5B that the barrier sleeve 260 is mounted over a portion of the external luer portion 132 and the rearward portion of the needle cover 250, such that the inner surface 286 of the barrier sleeve 260 engages the outer circumference of the external luer portion 132 in a pressure-fit contamination-barrier providing manner. Further, the protrusion 290 of the barrier sleeve 260 engages the rearward portion of the main sleeve 262 in a pressure-fit contamination-barrier providing manner. The rearward end of the barrier sleeve 260 is preferably disposed adjacent the forward end 104 of the syringe barrel 102 and the forward end 282 of the barrier sleeve 260 is preferably disposed adjacent the rearward end 266 of the main sleeve 262 of the needle cover 250.

Entrance of contaminants between the needle cover 250 and the barrier sleeve 260 is prevented due to the pressure-fit engagement between the protrusion 290 of the barrier sleeve 260 and the rearward portion of the needle cover 250 and due to pressure-fit engagement between the inner surface 286 of the barrier sleeve 260 and the outer circumference of the external luer portion 132, thereby sterile environment is provided within the inner volume of the needle cover 250 as long as the barrier sleeve 260 is coupled to the needle cover 250. The container closure integrity is thereby maintained as long as the barrier sleeve 260 is coupled to the needle cover 250.

These pressure-fit engagements provide for contamination-barrier for the inner volume of the needle cover 250, the inner volume of the needle 122, the inner volume of sleeve 260, the inner volume of the luer portion 110 and the inner volume of the syringe barrel 102 defined by the piston 136, all of these volumes are configured to fluidly communicate with each other.

Contamination-barrier is provided for prohibiting entrance of debris, harmful bacteria, viruses, other microorganisms, or any foreign particulates from the environment.

Upon removal of the needle cover 250, the forwardly facing shoulder 270 of the needle cover 250 engages the protrusion 290 of the barrier sleeve 260 and thereby pulls the barrier sleeve 260 along with the needle cover 250 and thus detaches the barrier sleeve 260 from the external luer portion 132. Upon removal of the needle cover 250, the barrier sleeve 260 remains coupled to the needle cover 250.

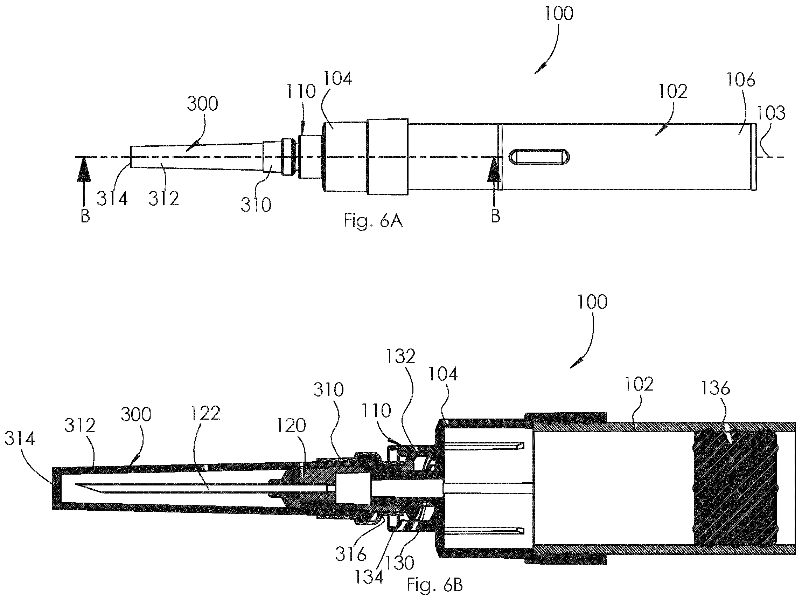

Reference is now made to Figs. 6A and 6B, which are respectively simplified planar side view and partial sectional view taken along lines B - B in Fig. 6A of a medical container with a covered needle attached thereto, constructed and operative in accordance with a sixth embodiment of the present invention.

As seen in Figs. 6A & 6B, a medical container 100 preferably includes a syringe barrel 102 arranged along a longitudinal axis 103 and having a forward end 104 and a rearward end 106. A luer portion 110 is formed at the forward end 104 of the syringe barrel 102 and extends longitudinally forwardly therefrom. A needle hub 120 is attached to the luer portion 110 and a needle 122 is fixedly attached to the needle hub 120.

It is noted that the medical container 100 may be any container known in the art, such as a pre-filled syringe, in which case the needle 122 is fixedly attached to the luer portion 110 of the container 100 and thus the luer portion 110 serves as the needle hub 120. Alternatively, the medical container 100 may be a cartridge with a luer portion 110 whereas the needle hub 120 with the needle 122 is attached to the luer portion 110.

It is appreciated that medical container 100 can be any type of conventional container, such as a cartridge commercially available from Schott Pharmaceutical Systems, Mainz, Germany or Vetter Pharma International USA Inc., IL, USA or Nuova Ompi S.r.l., Padua, Italy or may be any other suitable syringe or cartridge.

The luer portion 110 in accordance with the sixth embodiment of the present invention includes a luer lock, which has an inner luer portion 130 and an external luer portion 132, which generally surrounds the inner luer portion 130 and has an internal threading 134. In accordance with this embodiment of the present invention, the needle hub 120 is threadably attached to the luer portion 110 in a sealing manner, thereby providing sealing relationship between the needle hub 120 and the inner luer portion 130. It is noted that the luer portion 110 may alternatively include a luer slip, where no external luer portion, such as portion 132, is provided.

It is noted that alternatively another type of connector may be provided at the forward end 104 of the syringe barrel 102, instead of the luer portion 110, such as a tube connector or any other mechanical connector.

It is noted that further alternatively, the luer portion and the needle hub may be obviated and the needle can be fixedly attached directly to the forward end of the medical container 100, which serves as the syringe outlet portion. The needle 122 may be fixed to the syringe outlet portion using an adhesive for example or any other kind of permanent attachment to the syringe outlet portion.

The luer portion 110 may be an integral portion of the medical container 100 or may be attached to the front end 104 thereof.

It is also seen in Fig. 6B that at least one piston 136 is contained within the medical container 100 and adapted for slidable axial displacement relative to the syringe barrel 102. It is appreciated that the syringe barrel 102 may contain a single substance contained by a single piston or several substances, each one contained between a pair of pistons. The at least one piston 136 may also define a fluid chamber to be filled following the process of reconstitution or mixing of several substances, as particularly seen in the embodiment shown in Figs. 6A & 6B.

It is a particular feature of an embodiment of the present invention that a needle cover 300 is removably coupled to the needle hub 120 and a barrier sleeve 310, that is formed of a shrink sleeve, is mounted over a portion of the needle hub 120 and a rearward portion 302 of the needle cover 300 in a contamination-barrier providing manner.

It is specifically seen in Fig. 6B that the needle cover 300 has a generally conical main sleeve 312, having a closed forward end 314 and an open rearward end 316. It is noted that the barrier sleeve 310 is mounted over a portion of the needle hub 120 and the rearward portion of the needle cover 300, adjacent the rearward end 316.

It is noted that the barrier sleeve 310 is preferably made of a resilient material, such as FEP, PE, PVC or PTFE, for example. and it is configured to be attached to the needle hub 120 and to the needle cover 300 prior to the connection of the needle hub 120 to the luer portion 110. The attachment of the barrier sleeve 310 is preferably performed upon application of heat onto the barrier sleeve 310.

It is seen in Fig. 6B that the barrier sleeve 310 in accordance with the sixth embodiment of the present invention is mounted over the needle hub 120 and the needle cover 300 in a contamination-barrier providing manner. Entrance of contaminants between the needle cover 300 and the needle hub 120 is prevented due to attachment of the barrier sleeve 310 therebetween and thereby sterile environment is provided within the inner volume of the needle cover 300 as long as the barrier sleeve 310 is coupled to the needle cover 300 and to the needle hub 120. The container closure integrity is thereby maintained as long as the barrier sleeve 310 is coupled to the needle cover 300 and to the needle hub 120.

These engagement between the shrink sleeve 310, the needle cover 300 and the needle hub 120 provides for contamination-barrier for the inner volume of the needle cover 300, the inner volume of the needle 122, the inner volume of the inner luer portion 130 and the inner volume of the syringe barrel 102 defined by the piston 136, all of these volumes are configured to fluidly communicate with each other.

Contamination-barrier is provided for prohibiting entrance of debris, harmful bacteria, viruses, other microorganisms, or any foreign particulates from the environment.

Upon removal of the needle cover 300, the barrier sleeve 310 is preferably torn, alternatively the barrier sleeve 310 may remain attached to the needle cover 300 or to the needle hub 120.

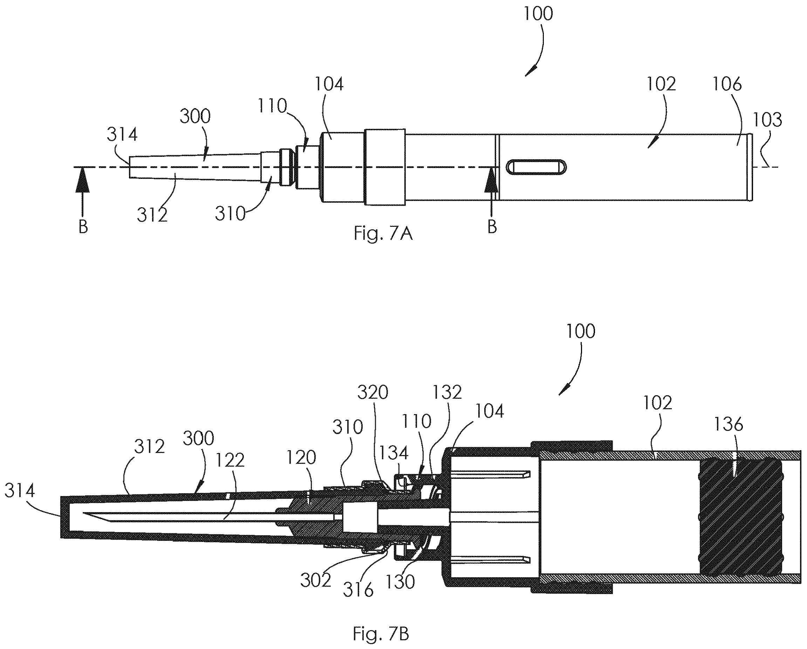

Reference is now made to Figs. 7A and 7B, which are respectively simplified planar side view and partial sectional view taken along lines B - B in Fig. 7A of a medical container with a covered needle attached thereto, constructed and operative in accordance with a seventh embodiment of the present invention.

The needle cover 300 and the barrier sleeve 310 in accordance with this seventh embodiment of the present invention are preferably identical in all respects to needle cover 300 and barrier sleeve 310 described in reference to the sixth embodiment illustrated in Figs. 6A and 6B, except that a barrier ring 320 is disposed rearwardly of the rearward end 316 of the needle cover 300. The barrier ring 320 is mounted onto the needle hub 120 and is located between the needle hub 120 and the barrier sleeve 310 for better providing contamination-barrier for the inner volume of the needle cover 300.

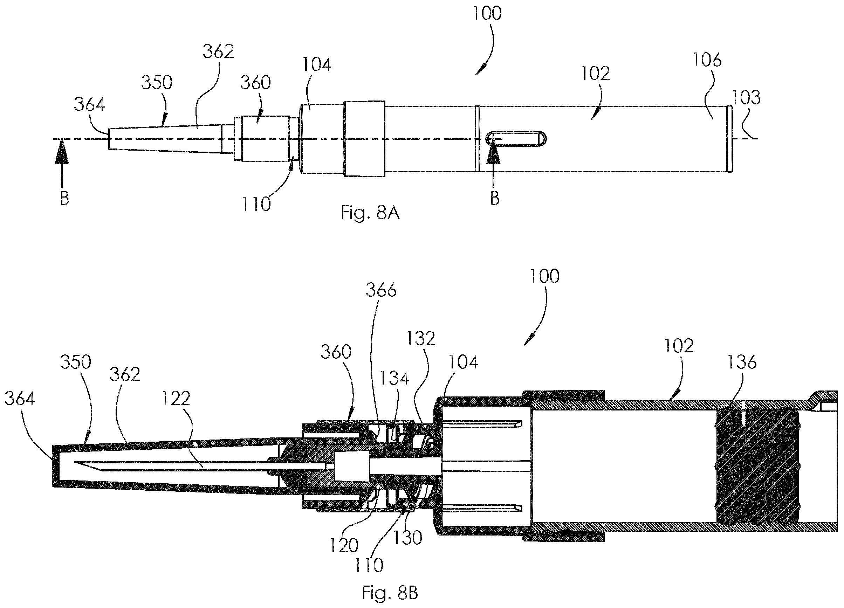

Reference is now made to Figs. 8A and 8B, which are respectively simplified planar side view and partial sectional view taken along lines B - B in Fig. 8A of a medical container with a covered needle attached thereto, constructed and operative in accordance with an eighth embodiment of the present invention.

As seen in Figs. 8A & 8B, a medical container 100 preferably includes a syringe barrel 102 arranged along a longitudinal axis 103 and having a forward end 104 and a rearward end 106. A luer portion 110 is formed at the forward end 104 of the syringe barrel 102 and extends longitudinally forwardly therefrom. A needle hub 120 is attached to the luer portion 110 and a needle 122 is fixedly attached to the needle hub 120.

It is noted that the medical container 100 may be any container known in the art, such as a pre-filled syringe, in which case the needle 122 is fixedly attached to the luer portion 110 of the container 100 and thus the luer portion 110 serves as the needle hub 120. Alternatively, the medical container 100 may be a cartridge with a luer portion 110 whereas the needle hub 120 with the needle 122 is attached to the luer portion 110.

It is appreciated that medical container 100 can be any type of conventional container, such as a cartridge commercially available from Schott Pharmaceutical Systems, Mainz, Germany or Vetter Pharma International USA Inc., IL, USA or Nuova Ompi S.r.l., Padua, Italy or may be any other suitable syringe or cartridge.

The luer portion 110 in accordance with the eighth embodiment of the present invention includes a luer lock, which has an inner luer portion 130 and an external luer portion 132, which generally surrounds the inner luer portion 130 and has an internal threading 134. In accordance with this embodiment of the present invention, the needle hub 120 is threadably attached to the luer portion 110 in a sealing manner, thereby providing sealing relationship between the needle hub 120 and the inner luer portion 130. It is noted that the luer portion 110 may alternatively include a luer slip, where no external luer portion, such as portion 132, is provided.

It is noted that alternatively another type of connector may be provided at the forward end 104 of the syringe barrel 102, instead of the luer portion 110, such as a tube connector or any other mechanical connector.

It is noted that further alternatively, the luer portion and the needle hub may be obviated and the needle can be fixedly attached directly to the forward end of the medical container 100, which serves as the syringe outlet portion. The needle 122 may be fixed to the syringe outlet portion using an adhesive for example or any other kind of permanent attachment to the syringe outlet portion.

The luer portion 110 may be an integral portion of the medical container 100 or may be attached to the front end 104 thereof.

It is also seen in Fig. 8B that at least one piston 136 is contained within the medical container 100 and adapted for slidable axial displacement relative to the syringe barrel 102. It is appreciated that the syringe barrel 102 may contain a single substance contained by a single piston or several substances, each one contained between a pair of pistons. The at least one piston 136 may also define a fluid chamber to be filled following the process of reconstitution or mixing of several substances, as particularly seen in the embodiment shown in Figs. 8A & 8B.

It is a particular feature of an embodiment of the present invention that a needle cover 350 is removably coupled to the needle hub 120 and a barrier sleeve 360, formed as a shrink sleeve, is mounted over the luer portion 110 and the rearward end of the needle cover 350 in a contamination-barrier providing manner. It is specifically seen in Fig. 8B that the needle cover 350 has a generally conical main sleeve 362, having a closed forward end 364 and an open rearward end 366. It is noted that the barrier sleeve 360 is mounted over the external luer portion 132 and the rearward portion of the needle cover 350, adjacent the rearward end 366.

It is noted that the barrier sleeve 360 is preferably made of a resilient material, such as FEP, PE, PVC or PTFE, for example. and it is configured to be attached to the luer portion 110 and to the needle cover 350 following connection of the needle hub 120 to the luer portion 110. The attachment of the barrier sleeve 360 is performed upon application of heat onto the barrier sleeve 360.

It is seen in Fig. 8B that the barrier sleeve 360 in accordance with the eighth embodiment of the present invention is mounted over the external luer portion 132 and the needle cover 350 in a contamination-barrier providing manner. Entrance of contaminants between the needle cover 350 and the needle hub 120 is prevented due to attachment of the barrier sleeve 360 to the needle cover 350 and the luer portion 110, and thereby sterile environment is provided within the inner volume of the needle cover 350 as long as the barrier sleeve 360 is coupled to the needle cover 350 and to the luer portion 110. The container closure integrity is thereby maintained as long as the barrier sleeve 360 is coupled to the needle cover 350 and to the luer portion 110.

The contamination-barrier providing engagement between the barrier sleeve 360, needle cover 350 and the external luer portion 132 provide for contamination-barrier for the inner volume of the needle cover 350, the inner volume of the needle 122, the inner volume of sleeve 360, the inner volume of the luer portion 110 and the inner volume of the syringe barrel 102 defined by the piston 136, all of these volumes are configured to fluidly communicate with each other.

Contamination-barrier is provided for prohibiting entrance of debris, harmful bacteria, viruses, other microorganisms, or any foreign particulates from the environment.

Upon removal of the needle cover 350, the barrier sleeve 360 is preferably torn, alternatively the barrier sleeve 360 may remain attached to the needle cover 350 or to the external luer portion 132 of the luer portion 110.

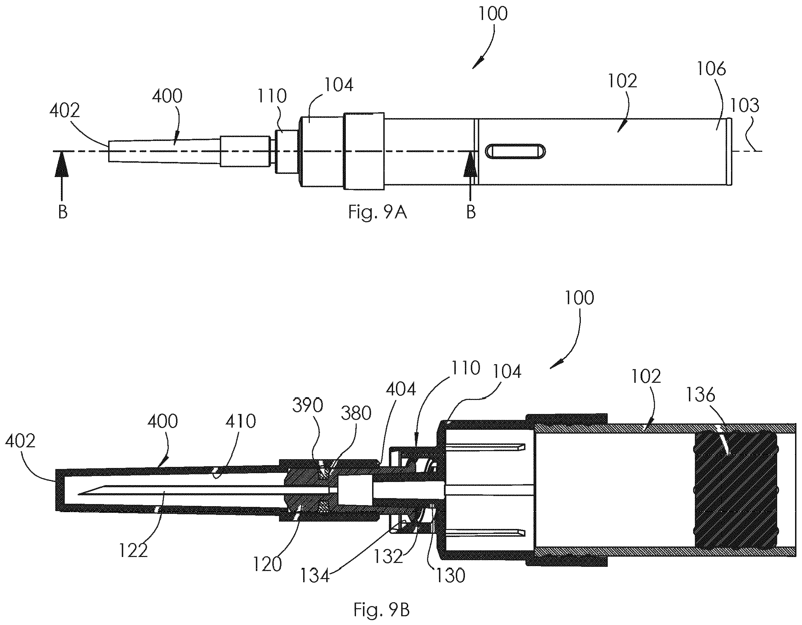

Reference is now made to Figs. 9A and 9B, which are respectively simplified planar side view and partial sectional view taken along lines B - B in Fig. 9A of a medical container with a covered needle attached thereto, constructed and operative in accordance with a ninth embodiment of the present invention.

As seen in Figs. 9A & 9B, a medical container 100 preferably includes a syringe barrel 102 arranged along a longitudinal axis 103 and having a forward end 104 and a rearward end 106. A luer portion 110 is formed at the forward end 104 of the syringe barrel 102 and extends longitudinally forwardly therefrom. A needle hub 120 is attached to the luer portion 110 and a needle 122 is fixedly attached to the needle hub 120.

As seen in Fig. 9B, it is a particular feature of this embodiment of the present invention that a groove 380 is formed on the outer circumference of the needle hub 120 and a barrier element 390 is inserted into the groove 380 and extends slightly radially outwardly from the outer circumference of the needle hub 120. Alternatively, the groove 380 may be obviated and the barrier element 390 may be mounted onto the outer circumference of the needle hub 120 either via pressure-fit or integrally molded therewith. The barrier element 390 is preferably made of a resilient material, such as silicon or TPE.

It is noted that the medical container 100 may be any container known in the art, such as a pre-filled syringe, in which case the needle 122 is fixedly attached to the luer portion 110 of the container 100 and thus the luer portion 110 serves as the needle hub 120. Alternatively, the medical container 100 may be a cartridge with a luer portion 110 whereas the needle hub 120 with the needle 122 is attached to the luer portion 110.

It is appreciated that medical container 100 can be any type of conventional container, such as a cartridge commercially available from Schott Pharmaceutical Systems, Mainz, Germany or Vetter Pharma International USA Inc., IL, USA or Nuova Ompi S.r.l., Padua, Italy or may be any other suitable syringe or cartridge.

The luer portion 110 in accordance with the ninth embodiment of the present invention includes a luer lock, which has an inner luer portion 130 and an external luer portion 132, which generally surrounds the inner luer portion 130 and has an internal threading 134. In accordance with this embodiment of the present invention, the needle hub 120 is threadably attached to the luer portion 110 in a sealing manner, thereby providing sealing relationship between the needle hub 120 and the inner luer portion 130. It is noted that the luer portion 110 may alternatively include a luer slip, where no external luer portion, such as portion 132, is provided.

It is noted that alternatively another type of connector may be provided at the forward end 104 of the syringe barrel 102, instead of the luer portion 110, such as a tube connector or any other mechanical connector.

It is noted that further alternatively, the luer portion and the needle hub may be obviated and the needle can be fixedly attached directly to the forward end of the medical container 100, which serves as the syringe outlet portion. The needle 122 may be fixed to the syringe outlet portion using an adhesive for example or any other kind of permanent attachment to the syringe outlet portion.

The luer portion 110 may be an integral portion of the medical container 100 or may be attached to the front end 104 thereof.

It is also seen in Fig. 9B that at least one piston 136 is contained within the medical container 100 and adapted for slidable axial displacement relative to the syringe barrel 102. It is appreciated that the syringe barrel 102 may contain a single substance contained by a single piston or several substances, each one contained between a pair of pistons. The at least one piston 136 may also define a fluid chamber to be filled following the process of reconstitution or mixing of several substances, as particularly seen in the embodiment shown in Figs. 9A & 9B.

It is a particular feature of an embodiment of the present invention that a needle cover 400 is removably coupled to the needle hub 120 in a contamination-barrier providing manner. The needle cover 400 has a closed forward end 402 and an open rearward end 404. The needle cover 400 defines an inner surface 410.

It is a particular feature of an embodiment of the present invention, as seen in Fig. 9B, that in accordance with the ninth embodiment of the present invention the barrier element 390, integrally formed with or attached to the needle hub 120, is adapted for engagement with the inner surface 410 of the needle cover 400 in a pressure-fit contamination-barrier providing manner, thereby preventing from any contaminants to enter between the needle hub 120 and the needle cover 400 and thereby provides for sterile environment within the inner volume of the needle cover 400 as long as it is coupled to the needle hub 120. The container closure integrity is thereby maintained as long as the needle cover 400 is coupled to the needle hub 120.

This pressure-fit engagement provides for contamination-barrier for the inner volume of the needle cover 400, the inner volume of the needle 122, the inner volume of the inner portion 132 of the luer portion 110 and the inner volume of the syringe barrel 102 defined by the piston 136, all of these volumes are configured to fluidly communicate with each other.

Contamination-barrier is provided for prohibiting entrance of debris, harmful bacteria, viruses, other microorganisms, or any foreign particulates from the environment.

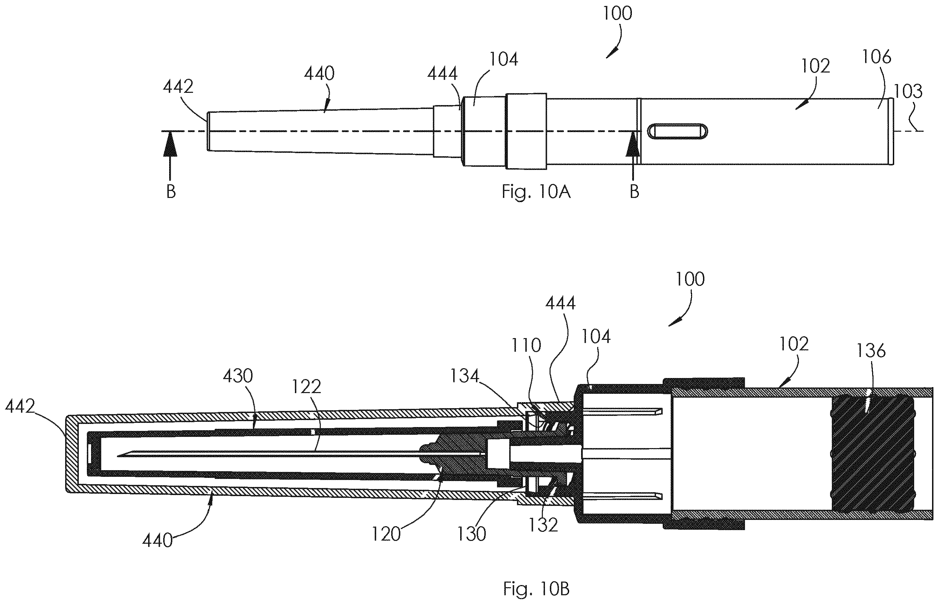

Reference is now made to Figs. 10A and 10B, which are respectively simplified planar side view and partial sectional view taken along lines B - B in Fig. 10A of a medical container with a covered needle attached thereto, constructed and operative in accordance with a tenth embodiment of the present invention.

As seen in Figs. 10A & 10B, a medical container 100 preferably includes a syringe barrel 102 arranged along a longitudinal axis 103 and having a forward end 104 and a rearward end 106. A luer portion 110 is formed at the forward end 104 of the syringe barrel 102 and extends longitudinally forwardly therefrom. A needle hub 120 is attached to the luer portion 110 and a needle 122 is fixedly attached to the needle hub 120.

It is noted that the medical container 100 may be any container known in the art, such as a pre-filled syringe, in which case the needle 122 is fixedly attached to the luer portion 110 of the container 100 and thus the luer portion 110 serves as the needle hub 120. Alternatively, the medical container 100 may be a cartridge with a luer portion 110 whereas the needle hub 120 with the needle 122 is attached to the luer portion 110.

It is appreciated that medical container 100 can be any type of conventional container, such as a cartridge commercially available from Schott Pharmaceutical Systems, Mainz, Germany or Vetter Pharma International USA Inc., IL, USA or Nuova Ompi S.r.l., Padua, Italy or may be any other suitable syringe or cartridge.

The luer portion 110 in accordance with the tenth embodiment of the present invention includes a luer lock, which has an inner luer portion 130 and an external luer portion 132, which generally surrounds the inner luer portion 130 and has an internal threading 134. In accordance with this embodiment of the present invention, the needle hub 120 is threadably attached to the luer portion 110 in a sealing manner, thereby providing sealing relationship between the needle hub 120 and the inner luer portion 130. It is noted that the luer portion 110 may alternatively include a luer slip, where no external luer portion, such as portion 132, is provided.

It is noted that alternatively another type of connector may be provided at the forward end 104 of the syringe barrel 102, instead of the luer portion 110, such as a tube connector or any other mechanical connector.

It is noted that further alternatively, the luer portion and the needle hub may be obviated and the needle can be fixedly attached directly to the forward end of the medical container 100, which serves as the syringe outlet portion. The needle 122 may be fixed to the syringe outlet portion using an adhesive for example or any other kind of permanent attachment to the syringe outlet portion.

The luer portion 110 may be an integral portion of the medical container 100 or may be attached to the front end 104 thereof.

It is also seen in Fig. 10B that at least one piston 136 is contained within the medical container 100 and adapted for slidable axial displacement relative to the syringe barrel 102. It is appreciated that the syringe barrel 102 may contain a single substance contained by a single piston or several substances, each one contained between a pair of pistons. The at least one piston 136 may also define a fluid chamber to be filled following the process of reconstitution or mixing of several substances, as particularly seen in the embodiment shown in Figs. 10A & 10B.

It is a particular feature of an embodiment of the present invention that a needle cover 430 is removably coupled to the needle hub 120 and a barrier cap 440 is mounted over the needle cover 430 and over the external luer portion 132 of the luer portion 110 in a contamination-barrier providing manner. The barrier cap 440 has a closed forward end 442 and an open rearward end 444.

It is noted that in accordance with an embodiment of the present invention, the needle sub-assembly comprising the needle hub 120 with the needle 122 covered by the needle cover 430 can be any type of conventional needle sub-assembly, such as the needle sub-assembly commercially available from Medicpro, Chungcheongnam-do, Korea or Medi-plus, New Jersey, United States or may be any other suitable needle sub-assembly.

It is noted that the barrier cap 440 is preferably made of a resilient material, such as silicon or TPE and it is configured to be attached to the luer portion 110 after connection of the needle hub 120 to the luer portion 110.

It is seen in Fig. 10B that in accordance with the tenth embodiment of the present invention that the inner circumference of the rearward portion of the needle cover 430 engages the outer circumference of the needle hub 120. The barrier cap 440 is mounted over the needle cover 430 and over the luer portion 110 in a pressure-fit contamination-barrier providing manner. The forward end 442 of the barrier cap 440 is preferably disposed slightly forwardly of the forward end of the needle cover 430 and the rearward end 444 of the barrier cap 440 is generally disposed adjacent the forward end 104 of the syringe barrel 102. The rearward portion of the barrier cap 440 is mounted over the external luer portion 132 in a pressure-fit manner, such that the inner circumference of the rearward portion of the barrier cap 440 engages the outer circumference of the external luer portion 132 of the luer portion 110 in a pressure-fit manner, thereby preventing from any contaminants to enter between the barrier cap 440 and the luer portion 110 and thereby provides for sterile environment within the inner volume of the needle cover 430 as long as the barrier cap 440 is coupled to the luer portion 110. The container closure integrity is thereby maintained as long as the barrier cap 440 is coupled to the luer portion 110.

This pressure-fit engagements provides for contamination-barrier providing for the inner volume of barrier cap 440, needle cover 430, the inner volume of the needle 122, the inner volume of the luer portion 110 and the inner volume of the syringe barrel 102 defined by the piston 136, all of these volumes are configured to fluidly communicate with each other.

Contamination-barrier is provided for prohibiting entrance of debris, harmful bacteria, viruses, other microorganisms, or any foreign particulates from the environment.

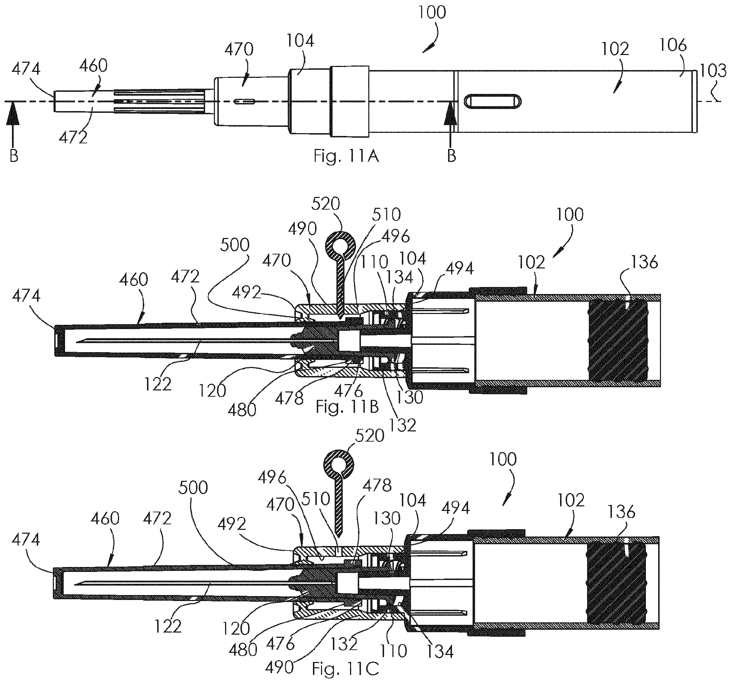

Reference is now made to Figs. 11A, 11B and 11C, which are respectively simplified planar side view, a partial sectional view taken along lines B - B in Fig. 11A of a medical container with a covered needle attached thereto showing the device in a storage operative orientation and a partial sectional view taken along lines B - B in Fig. 11A of a medical container with a covered needle attached thereto showing the device in use operative orientation, constructed and operative in accordance with an eleventh embodiment of the present invention.

As seen in Figs. 11A - 11C, a medical container 100 preferably includes a syringe barrel 102 arranged along a longitudinal axis 103 and having a forward end 104 and a rearward end 106. A luer portion 110 is formed at the forward end 104 of the syringe barrel 102 and extends longitudinally forwardly therefrom. A needle hub 120 is attached to the luer portion 110 and a needle 122 is fixedly attached to the needle hub 120.

It is noted that the medical container 100 may be any container known in the art, such as a pre-filled syringe, in which case the needle 122 is fixedly attached to the luer portion 110 of the container 100 and thus the luer portion 110 serves as the needle hub 120. Alternatively, the medical container 100 may be a cartridge with a luer portion 110 whereas the needle hub 120 with the needle 122 is attached to the luer portion 110.

It is appreciated that medical container 100 can be any type of conventional container, such as a cartridge commercially available from Schott Pharmaceutical Systems, Mainz, Germany or Vetter Pharma International USA Inc., IL, USA or Nuova Ompi S.r.l., Padua, Italy or may be any other suitable syringe or cartridge.

The luer portion 110 in accordance with the eleventh embodiment of the present invention includes a luer lock, which has an inner luer portion 130 and an external luer portion 132, which generally surrounds the inner luer portion 130 and has an internal threading 134. In accordance with this embodiment of the present invention, the needle hub 120 is threadably attached to the luer portion 110 in a sealing manner, thereby providing sealing relationship between the needle hub 120 and the inner luer portion 130. It is noted that the luer portion 110 may alternatively include a luer slip, where no external luer portion, such as portion 132, is provided.

It is noted that alternatively another type of connector may be provided at the forward end 104 of the syringe barrel 102, instead of the luer portion 110, such as a tube connector or any other mechanical connector.

It is noted that further alternatively, the luer portion and the needle hub may be obviated and the needle can be fixedly attached directly to the forward end of the medical container 100, which serves as the syringe outlet portion. The needle 122 may be fixed to the syringe outlet portion using an adhesive for example or any other kind of permanent attachment to the syringe outlet portion.

The luer portion 110 may be an integral portion of the medical container 100 or may be attached to the front end 104 thereof.

It is also seen in Figs. 11B & 11C that at least one piston 136 is contained within the medical container 100 and adapted for slidable axial displacement relative to the syringe barrel 102. It is appreciated that the syringe barrel 102 may contain a single substance contained by a single piston or several substances, each one contained between a pair of pistons. The at least one piston 136 may also define a fluid chamber to be filled following the process of reconstitution or mixing of several substances, as particularly seen in the embodiment shown in Figs. 11A - 11C.

It is a particular feature of an embodiment of the present invention that a needle cover 460 is removably coupled to the needle hub 120 and a barrier sleeve 470 is mounted over the luer portion 110 and the rearward end of the needle cover 460 in a pressure-fit contamination-barrier providing manner.

It is noted that in accordance with an embodiment of the present invention, the needle sub-assembly comprising the needle hub 120 with the needle 122 covered by the needle cover 460 can be any type of conventional needle sub-assembly, such as the needle sub-assembly commercially available from Medicpro, Chungcheongnam-do, Korea or Medi-plus, New Jersey, United States or may be any other suitable needle sub-assembly.

It is specifically seen in Figs. 11B & 11C that the needle cover 460 has a generally conical main sleeve 472, having a closed forward end 474 and an open rearward end 476. An enlarged generally annular portion 478 is formed at the rearward end 476 of the needle cover 460 and forms a forwardly facing shoulder 480.

It is also seen in Figs. 11B & 11C that the barrier sleeve 470 has a hollow cylindrical portion 490, a forward end 492 and a rearward end 494. The barrier sleeve 470 defines an inner surface 496. A protrusion 500 extends radially inwardly from the inner surface 496 of the barrier sleeve 470, and is preferably formed at a location adjacent the forward end 492 of the barrier sleeve 470. Alternatively, the barrier sleeve 470 may have a cylindrical inner circumference.

It is a particular feature of an embodiment of the present invention that at least one aperture 510 is formed through the side wall of the barrier sleeve 470. It is particularly seen in Fig. 11B, where the device is shown in the storage operative orientation, that a removeable pin 520 is partially inserted through the aperture 510 of the barrier sleeve 470 and thus contributes to contamination-barrier providing for the inner volume of the barrier sleeve 470, the inner volume of the needle cover 460, the inner volume of needle 122, the inner volume of the luer portion 110 and the inner volume of the syringe barrel 102 defined by piston 136 and also selectably seals the passage of fluid between the atmosphere and the inner volume of the barrier sleeve 470.

Contamination-barrier is provided for prohibiting entrance of debris, harmful bacteria, viruses, other microorganisms, or any foreign particulates from the environment.

It is noted that the barrier sleeve 470 is preferably made of a resilient material, such as silicon or TPE and it is configured to be attached to the luer portion 110 after connection of the needle hub 120 to the luer portion 110.

It is seen in Figs. 11B & 11C that the needle cover 460 in accordance with the eleventh embodiment of the present invention is mounted over the needle hub 120, such that the inner circumference of the rearward portion of the needle cover 460 engages the outer circumference of the needle hub 120. It is further particularly seen in Figs. 11B & 11C that the barrier sleeve 470 is mounted over a portion of the external luer portion 132 and the rearward portion of the needle cover 460, such that the inner surface 496 of the barrier sleeve 470 engages the outer circumference of the external luer portion 132 in a pressure-fit contamination-barrier providing manner. Further, the protrusion 500 of the barrier sleeve 470 engages the rearward portion of the main sleeve 472 in a pressure-fit contamination-barrier providing manner. The rearward end 494 of the barrier sleeve 470 is preferably disposed adjacent the forward end 104 of the syringe barrel 102 and the forward end 492 of the barrier sleeve 470 is preferably disposed adjacent the rearward end 476 of the main sleeve 472 of the needle cover 460.

Fig. 11B particularly illustrates the device in storage operative orientation, where the removeable pin 520 is within the aperture 510 of the barrier sleeve 470, thus preventing fluid passage between the atmosphere and the inner volume of the barrier sleeve 470.

Fig. 11C particularly illustrates the device in use operative orientation, where the removeable pin 520 is removed from aperture 510 of the barrier sleeve 470, thus provides for venting of the barrier sleeve 470 by allowing fluid passage between the atmosphere and the inner volume of the barrier sleeve 470. This venting prevents build-up of pressure between the barrier sleeve 470, needle cover 460 and luer portion 110, which can lead to inadvertent displacement of the piston 136 within the syringe barrel 102 or inadvertent removal of the barrier sleeve 470 from the luer portion 110 or from the needle cover 460 due to pressure build-up during reconstitution of the medicament within the syringe barrel 102 or due to pressure differences during transportation of the medical container 100.

It is noted that the removable pin 520 may be removed by the user or it maybe removed automatically by attaching the removeable pin 520 to the packaging of the syringe and the removal of the removeable pin 520 is then affected automatically during opening of the packaging.

It is a particular feature of an embodiment of the present invention that entrance of contaminants between the needle cover 460 and the barrier sleeve 470 is prevented both due to the pressure-fit engagement between the protrusion 500 of the barrier sleeve 470 and the rearward portion of the needle cover 460, and due to pressure-fit engagement of the rearward end of the barrier sleeve 470 with the external portion 132 of the luer portion 110 and due to insertion of the removeable pin 520 through aperture 510 in the barrier sleeve 470, as seen in Fig. 11B in the storage operative orientation of the device. Sterile environment is thereby provided within the inner volume of the needle cover 460 as long as the barrier sleeve 470 is coupled to the needle cover 460 and the removeable pin 520 is inserted into aperture 510, as seen in Fig. 11B. The container closure integrity is thereby maintained as long as the barrier sleeve 470 is coupled to the needle cover 460 and the removeable pin 520 is inserted into aperture 510.

These pressure-fit engagements provide for contamination-barrier for the inner volume of the needle cover 460, the inner volume of the needle 122, the inner volume of sleeve 470, the inner volume of the luer portion 110 and the inner volume of the syringe barrel 102 defined by the piston 136, all of these volumes are configured to fluidly communicate with each other.

Contamination-barrier is provided for prohibiting entrance of debris, harmful bacteria, viruses, other microorganisms, or any foreign particulates from the environment.

Upon removal of the needle cover 460, the forwardly facing shoulder 480 of the needle cover 460 engages the protrusion 500 of the barrier sleeve 470 and thereby pulls the barrier sleeve 470 along with the needle cover 460 and thus detaches the barrier sleeve 470 from the external luer portion 132. Upon removal of the needle cover 460, the barrier sleeve 470 remains coupled to the needle cover 460.

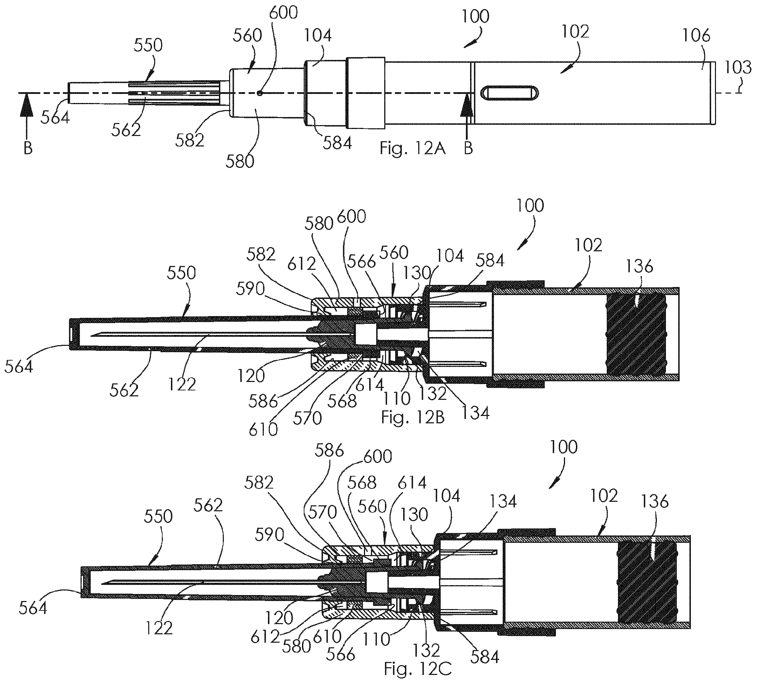

Reference is now made to Figs. 12A, 12B and 12C, which are respectively simplified planar side view, a partial sectional view taken along lines B - B in Fig. 12A of a medical container with a covered needle attached thereto showing the device in a storage operative orientation and a partial sectional view taken along lines B - B in Fig. 12A of a medical container with a covered needle attached thereto showing the device in use operative orientation, constructed and operative in accordance with a twelfth embodiment of the present invention.

As seen in Figs. 12A - 12C, a medical container 100 preferably includes a syringe barrel 102 arranged along a longitudinal axis 103 and having a forward end 104 and a rearward end 106. A luer portion 110 is formed at the forward end 104 of the syringe barrel 102 and extends longitudinally forwardly therefrom. A needle hub 120 is attached to the luer portion 110 and a needle 122 is fixedly attached to the needle hub 120.

It is noted that the medical container 100 may be any container known in the art, such as a pre-filled syringe, in which case the needle 122 is fixedly attached to the luer portion 110 of the container 100 and thus the luer portion 110 serves as the needle hub 120. Alternatively, the medical container 100 may be a cartridge with a luer portion 110 whereas the needle hub 120 with the needle 122 is attached to the luer portion 110.

It is appreciated that medical container 100 can be any type of conventional container, such as a cartridge commercially available from Schott Pharmaceutical Systems, Mainz, Germany or Vetter Pharma International USA Inc., IL, USA or Nuova Ompi S.r.l., Padua, Italy or may be any other suitable syringe or cartridge.

The luer portion 110 in accordance with the twelfth embodiment of the present invention includes a luer lock, which has an inner luer portion 130 and an external luer portion 132, which generally surrounds the inner luer portion 130 and has an internal threading 134. In accordance with this embodiment of the present invention, the needle hub 120 is threadably attached to the luer portion 110 in a sealing manner, thereby providing sealing relationship between the needle hub 120 and the inner luer portion 130. It is noted that the luer portion 110 may alternatively include a luer slip, where no external luer portion, such as portion 132, is provided.

It is noted that alternatively another type of connector may be provided at the forward end 104 of the syringe barrel 102, instead of the luer portion 110, such as a tube connector or any other mechanical connector.

It is noted that further alternatively, the luer portion and the needle hub may be obviated and the needle can be fixedly attached directly to the forward end of the medical container 100, which serves as the syringe outlet portion. The needle 122 may be fixed to the syringe outlet portion using an adhesive for example or any other kind of permanent attachment to the syringe outlet portion.

The luer portion 110 may be an integral portion of the medical container 100 or may be attached to the front end 104 thereof.

It is also seen in Figs. 12B & 12C that at least one piston 136 is contained within the medical container 100 and adapted for slidable axial displacement relative to the syringe barrel 102. It is appreciated that the syringe barrel 102 may contain a single substance contained by a single piston or several substances, each one contained between a pair of pistons. The at least one piston 136 may also define a fluid chamber to be filled following the process of reconstitution or mixing of several substances, as particularly seen in the embodiment shown in Figs. 12A - 12C.

It is a particular feature of an embodiment of the present invention that a needle cover 550 is removably coupled to the needle hub 120 and a barrier sleeve 560 is mounted over the luer portion 110 and the rearward end of the needle cover 550 in a pressure-fit contamination-barrier providing manner.

It is noted that in accordance with an embodiment of the present invention, the needle sub-assembly comprising the needle hub 120 with the needle 122 covered by the needle cover 550 can be any type of conventional needle sub-assembly, such as the needle sub-assembly commercially available from Medicpro, Chungcheongnam-do, Korea or Medi-plus, New Jersey, United States or may be any other suitable needle sub-assembly.

It is specifically seen in Figs. 12B & 12C that the needle cover 550 has a generally conical main sleeve 562, having a closed forward end 564 and an open rearward end 566. An enlarged generally annular portion 568 is formed at the rearward end 566 of the needle cover 550 and forms a forwardly facing shoulder 570.

It is also seen in Figs. 12B & 12C that the barrier sleeve 560 has a hollow cylindrical portion 580, a forward end 582 and a rearward end 584. The barrier sleeve 560 defines an inner surface 586. A protrusion 590 extends radially inwardly from the inner surface 586 of the barrier sleeve 560, and is preferably formed at a location adjacent the forward end 582 of the barrier sleeve 560. Alternatively, the barrier sleeve 560 may have a cylindrical inner circumference.

It is noted that the barrier sleeve 560 is preferably made of a resilient material, such as silicon or TPE and it is configured to be attached to the luer portion 110 after connection of the needle hub 120 to the luer portion 110.

It is a particular feature of an embodiment of the present invention that at least one aperture 600 is formed through the side wall of the barrier sleeve 560. It is particularly seen in Figs. 12B & 12C that a moveable ring 610 is slidably mounted over the needle cover 550 and is disposed between the needle cover 550 and the barrier sleeve 560 and is configured to divide the inner volume of the barrier sleeve 560 into two chambers, a forward chamber 612 and a rearward chamber 614.

It is a further particular feature of an embodiment of the present invention that the moveable ring 610 is moveable relative to the needle cover 550 and relative to the barrier sleeve 560 upon pressure change within the inner volume of the barrier sleeve 560.

It is particularly seen in Fig. 12B, where the device is shown in storage operative orientation, that the moveable ring 610 engages the forwardly facing shoulder 570 of the needle cover 550 and is axially aligned with aperture 600 of the barrier sleeve 560, thus seals the passage of fluid between the atmosphere and the inner volume of the barrier sleeve 560. It is particularly noted that the moveable ring 610 contributes in contamination-barrier providing for the inner volume of the needle cover 550, the inner volume of the needle 122, the inner volume of sleeve 560, the inner volume of the luer portion 110 and the inner volume of the syringe barrel 102 defined by the piston 136, all of these volumes are configured to fluidly communicate with each other.

The forward chamber 612 is defined between the moveable ring 610 and the forward end 582 of the barrier sleeve 560 and the rearward chamber 614 is defined between the moveable ring 610 and the forward end 104 of the syringe barrel 102.

It is seen in Figs. 12B & 12C that the needle cover 550 in accordance with the twelfth embodiment of the present invention is mounted over the needle hub 120, such that the inner circumference of the rearward portion of the needle cover 550 engages the outer circumference of the needle hub 120. It is further particularly seen in Figs. 12B & 12C that the barrier sleeve 560 is mounted over a portion of the external luer portion 132 and the rearward portion of the needle cover 550, such that the inner surface 586 of the barrier sleeve 560 engages the outer circumference of the external luer portion 132 in a pressure-fit contamination-barrier providing manner. Further, the protrusion 590 of the barrier sleeve 560 engages the rearward portion of the main sleeve 562 in a pressure-fit contamination-barrier providing manner. The rearward end 584 of the barrier sleeve 560 is preferably disposed adjacent the forward end 104 of the syringe barrel 102 and the forward end 582 of the barrier sleeve 560 is preferably disposed adjacent the rearward end 566 of the main sleeve 562 of the needle cover 550.

These pressure-fit engagements provide for contamination-barrier for the inner volume of the needle cover 550, the inner volume of the needle 122, the inner volume of sleeve 560, the inner volume of the luer portion 110 and the inner volume of the syringe barrel 102 defined by the piston 136, all of these volumes are configured to fluidly communicate with each other.

Fig. 12B particularly illustrates the device in storage operative orientation, where the moveable ring 610 is aligned with aperture 600 of the barrier sleeve 560, thus preventing fluid passage between the atmosphere and the inner volume of the barrier sleeve 560 and contributes in contamination-barrier providing for the inner volume of the barrier sleeve 560 and the other components of the medical container 100, as described in detail hereinabove.

Contamination-barrier is provided for prohibiting entrance of debris, harmful bacteria, viruses, other microorganisms, or any foreign particulates from the environment.

Fig. 12C particularly illustrates the device in use operative orientation, where the moveable ring 610 is displaced from alignment with aperture 600 of the barrier sleeve 560, thus provides for venting of the barrier sleeve 560 by allowing fluid passage between the atmosphere and the inner volume of the barrier sleeve 560. It is particularly seen in Fig. 12C that the volume of the forward chamber 612 is decreased and the volume of the rearward chamber 614 increased respectively upon axial displacement of the moveable ring 610 relative to the barrier sleeve 560 and relative to the needle cover 550. This venting prevents build-up of pressure between the barrier sleeve 560, the needle cover 550 and the luer portion 110, which can lead to inadvertent displacement of the piston 136 within the syringe barrel 102 or inadvertent removal of the barrier sleeve 560 from the luer portion 110 or from the needle cover 550 due to pressure build-up during reconstitution of the medicament within the syringe barrel 102 or due to pressure differences during transportation of the medical container 100.

It is a particular feature of an embodiment of the present invention that during reconstitution of the medicament within the medical container 100, the forward displacement of the piston 136 increases the pressure within the rearward chamber 614 up to a point in which the pressure difference causes displacement of the moveable ring 6410 forwardly and out of alignment with aperture 600, thereby allowing for venting of the air pressure within the rearward chamber 614.

It is a particular feature of an embodiment of the present invention that entrance of contaminants between the needle cover 550 and the barrier sleeve 560 is prevented due to the pressure-fit engagement between the protrusion 590 of the barrier sleeve 560 and the rearward portion of the needle cover 550, due to pressure-fit engagement between the inner surface of the rearward end of the barrier sleeve 560 and the external luer portion 132 and due to sealing of the aperture 600 by the moveable ring 610, as seen in Fig. 12B in the storage operative orientation of the device. Sterile environment is thereby provided within the inner volume of the barrier sleeve 560, the luer portion 110, the needle cover 550 and the needle 122 as well as the inner volume of the syringe barrel 102 confined by the pistpn 136 as long as the barrier sleeve 560 is coupled to the needle cover 550 and to the external luer portion 132 and the moveable ring 610 seals the aperture 600 in the barrier sleeve 560, as seen in Fig. 12B. The container closure integrity is thereby maintained as long as the barrier sleeve 560 is coupled to the needle cover 550 and the moveable ring 560 seals the aperture 600 in the barrier sleeve 560.

Upon removal of the needle cover 550, the forwardly facing shoulder 570 of the needle cover 550 engages the protrusion 590 of the barrier sleeve 560 and thereby pulls the barrier sleeve 560 along with the needle cover 550 and thus detaches the barrier sleeve 560 from the external luer portion 132. Upon removal of the needle cover 550, the barrier sleeve 560 remains coupled to the needle cover 550.

It is noted that alternatively, the barrier sleeve 560 may be replaced by a barrier cap, similar to barrier cap 440, illustrated in Figs. 10A & 10B, which has an aperture through the side wall thereof and the moveable ring 610 is configured to seal the aperture within barrier cap 440 in storage operative orientation.

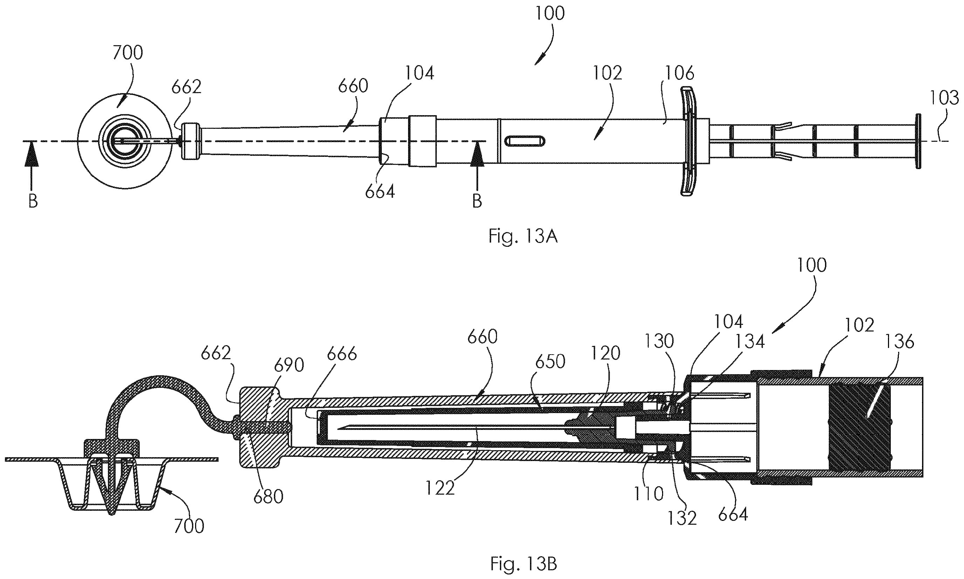

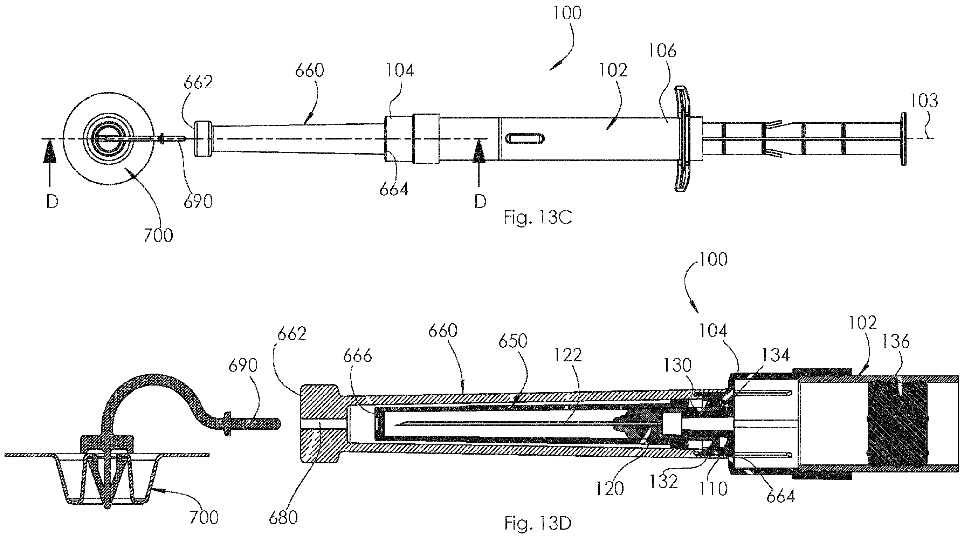

Reference is now made to Figs. 13A and 13B, which are respectively simplified planar side view and a partial sectional view taken along lines B - B in Fig. 13A of a medical container with a covered needle attached thereto showing the device in a storage operative orientation, constructed and operative in accordance with a thirteenth embodiment of the present invention. Reference is additionally made to Figs. 13C and 13D, which are respectively simplified planar side view and a partial sectional view taken along lines D - D in Fig. 13C of a medical container with a covered needle attached thereto showing the device in use operative orientation, constructed and operative in accordance with the thirteenth embodiment of the present invention.

As seen in Figs. 13A & 13B, a medical container 100 preferably includes a syringe barrel 102 arranged along a longitudinal axis 103 and having a forward end 104 and a rearward end 106. A luer portion 110 is formed at the forward end 104 of the syringe barrel 102 and extends longitudinally forwardly therefrom. A needle hub 120 is attached to the luer portion 110 and a needle 122 is fixedly attached to the needle hub 120.

It is noted that the medical container 100 may be any container known in the art, such as a pre-filled syringe, in which case the needle 122 is fixedly attached to the luer portion 110 of the container 100 and thus the luer portion 110 serves as the needle hub 120. Alternatively, the medical container 100 may be a cartridge with a luer portion 110 whereas the needle hub 120 with the needle 122 is attached to the luer portion 110.

It is appreciated that medical container 100 can be any type of conventional container, such as a cartridge commercially available from Schott Pharmaceutical Systems, Mainz, Germany or Vetter Pharma International USA Inc., IL, USA or Nuova Ompi S.r.l., Padua, Italy or may be any other suitable syringe or cartridge.

The luer portion 110 in accordance with the thirteenth embodiment of the present invention includes a luer lock, which has an inner luer portion 130 and an external luer portion 132, which generally surrounds the inner luer portion 130 and has an internal threading 134. In accordance with this embodiment of the present invention, the needle hub 120 is threadably attached to the luer portion 110 in a sealing manner, thereby providing sealing relationship between the needle hub 120 and the inner luer portion 130. It is noted that the luer portion 110 may alternatively include a luer slip, where no external luer portion, such as portion 132, is provided.

It is noted that alternatively another type of connector may be provided at the forward end 104 of the syringe barrel 102, instead of the luer portion 110, such as a tube connector or any other mechanical connector.

It is noted that further alternatively, the luer portion and the needle hub may be obviated and the needle can be fixedly attached directly to the forward end of the medical container 100, which serves as the syringe outlet portion. The needle 122 may be fixed to the syringe outlet portion using an adhesive for example or any other kind of permanent attachment to the syringe outlet portion.

The luer portion 110 may be an integral portion of the medical container 100 or may be attached to the front end 104 thereof.

It is also seen in Fig. 13B that at least one piston 136 is contained within the medical container 100 and adapted for slidable axial displacement relative to the syringe barrel 102. It is appreciated that the syringe barrel 102 may contain a single substance contained by a single piston or several substances, each one contained between a pair of pistons. The at least one piston 136 may also define a fluid chamber to be filled following the process of reconstitution or mixing of several substances, as particularly seen in the embodiment shown in Figs. 13A - 13D.

It is a particular feature of an embodiment of the present invention that a needle cover 650 is removably coupled to the needle hub 120 and a barrier cap 660 is mounted over the needle cover 650 and over the external luer portion 132 of the luer portion 110 in a contamination-barrier providing manner. The barrier cap 660 has a closed forward end 662 and an open rearward end 664. The needle cover 650 has a closed forward end 666 and an open rearward end 668.

It is noted that in accordance with an embodiment of the present invention, the needle sub-assembly comprising the needle hub 120 with the needle 122 covered by the needle cover 650 can be any type of conventional needle sub-assembly, such as the needle sub-assembly commercially available from Medicpro, Chungcheongnam-do, Korea or Medi-plus, New Jersey, United States or may be any other suitable needle sub-assembly.

It is seen in Fig. 13B that in accordance with the thirteenth embodiment of the present invention that the inner circumference of the rearward portion of the needle cover 650 engages the outer circumference of the needle hub 120. The barrier cap 660 is mounted over the needle cover 650. The barrier cap 660 is also mounted over the luer portion 110 in a pressure-fit contamination-barrier providing manner. The forward end 662 of the barrier cap 660 is preferably disposed slightly forwardly of the forward end 666 of the needle cover 650 and the rearward end 664 of the barrier cap 660 is generally disposed adjacent the forward end 104 of the syringe barrel 102. The rearward portion of the barrier cap 660 is mounted over the external luer portion 132 in a pressure-fit contamination-barrier providing manner, such that the inner circumference of the rearward portion of the barrier cap 660 engages the outer circumference of the external luer portion 132 of the luer portion 110, thereby preventing from any contaminants to enter between the barrier cap 660 and the luer portion 110 and thereby provides for sterile environment within the inner volume of the needle cover 650 as long as the barrier cap 660 is coupled to the luer portion 110. The container closure integrity is thereby maintained as long as the barrier cap 660 is coupled to the luer portion 110.

This pressure-fit engagements provides for contamination-barrier for the inner volume of the barrier cap 660, the inner volume of the needle cover 650, the inner volume of the needle 122, the inner volume of the luer portion 110 and the inner volume of the syringe barrel 102 defined by the piston 136, all of these volumes are configured to fluidly communicate with each other.

Contamination-barrier is provided for prohibiting entrance of debris, harmful bacteria, viruses, other microorganisms, or any foreign particulates from the environment.

It is a particular feature of an embodiment of the present invention that an aperture 680 is formed in the closed forward end 662 of the barrier cap 660. It is particularly seen in Figs. 13A & 13B, where the device is shown in storage operative orientation, that a removeable pin 690 is inserted through the aperture 680, thus sealing the passage of fluid between the atmosphere and the inner volume of the barrier cap 660 and contributing to providing a sterile environment within the inner volume of barrier cap 660, needle cover 650, luer portion 110, needle 122 and the inner volume of the syringe barrel 102 defined by the piston 136. It is also seen in Figs. 13A & 13B that the removeable pin 690 is fixedly attached to a packaging portion 700 of the device 100.

Figs. 13C & 13D particularly illustrate the device in use operative orientation, where the removeable pin 690 is removed from aperture 680 of the barrier cap 660, thus provides for venting of the barrier cap 660 by allowing fluid passage between the atmosphere and the inner volume of the barrier cap 660. This venting prevents build-up of pressure between the barrier cap 660, the needle cover 650 and the luer portion 110, which can lead to inadvertent displacement of the piston 136 within the syringe barrel 102 or inadvertent removal of the barrier cap 660 from the luer portion 110 due to pressure build-up during reconstitution of the medicament within the syringe barrel 102 or due to pressure differences during transportation of the medical container 100.

It is noted that the removable pin 690 is preferably removed automatically upon removal of the device 100 from the packaging 700, due to fixed attachment of the removeable pin 690 to the packaging 700.

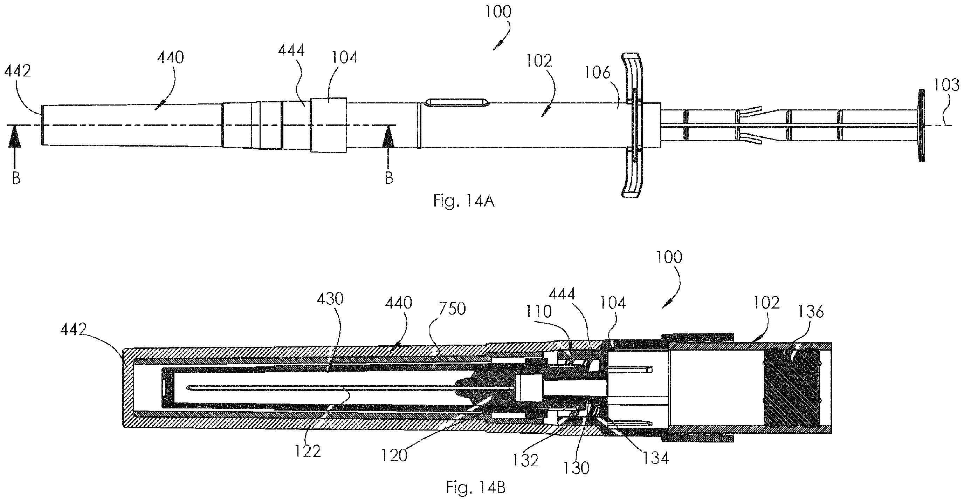

Reference is now made to Figs. 14A and 14B, which are respectively simplified planar side view and partial sectional view taken along lines B - B in Fig. 14A of a medical container with a covered needle attached thereto, constructed and operative in accordance with a fourteenth embodiment of the present invention.

The needle cover 430 and the barrier cap 440 in accordance with this fourteenth embodiment of the present invention are preferably identical in all respects to needle cover 430 and barrier cap 440 described in reference to the tenth embodiment illustrated in Figs. 10A and 10B, except that a rigid sleeve 750 is mounted between the needle cover 430 and the resilient barrier cap 440. The rigid sleeve 750 preferably extends substantially along the entire longitudinal extent of the needle cover 430. The inner circumference of the barrier cap 440 preferably engages the outer circumference of the rigid sleeve 750. The rigid sleeve 750 generally surrounds the needle cover 430.

It is noted that in accordance with an embodiment of the present invention, the needle sub-assembly comprising the needle hub 120 with the needle 122 covered by the needle cover 430 can be any type of conventional needle sub-assembly, such as the needle sub-assembly commercially available from Medicpro, Chungcheongnam-do, Korea or Medi-plus, New Jersey, United States or may be any other suitable needle sub-assembly.

It is a particular feature of an embodiment of the present invention that the pressure-fit engagement of the barrier cap 440 with the external luer portion 132 of the luer portion 110 is configured to provide contamination-barrier for the inner volume of the needle cover 430, the inner volume of the needle 122, the inner volume of the luer portion 110 and the inner volume of the syringe barrel 102 defined by the piston 136, all of these volumes are configured to fluidly communicate with each other. Addition of the rigid sleeve 750 provides for rigidity of the barrier cap 440 and prevents inadvertent removal of the needle cover 430 by the user upon pressing on the resilient barrier cap 440.

Contamination-barrier is provided for prohibiting entrance of debris, harmful bacteria, viruses, other microorganisms, or any foreign particulates from the environment.

It is a particular feature of an embodiment of the present invention that a porous barrier element may be mounted over a needle cover or a needle hub, alternatively the needle cover itself my be porous, such as to provide a contamination-barrier for the inner volume in which the needle is disposed and at the same time allow passage of gas from the environment.

It will be appreciated by persons skilled in the art that the present invention is not limited by what has been particularly shown and described hereinabove. Rather the scope of the present invention includes both combinations and sub-combinations of various features described hereinabove as well as variations and modifications thereof which are not in the prior art.

## Claims

1. A medical container with a needle situated within a volume enclosed in a contamination-barrier manner from the environment, comprising:
a syringe barrel with a piston slidably disposed therewithin;
said syringe having a syringe outlet portion at a forward end thereof;
said needle coupled with said syringe outlet portion;
a needle cover being removably coupled to said syringe outlet portion and defining said volume;
a barrier element coupled to said syringe outlet portion in a contamination-barrier providing manner, such that entrance of contaminants into said volume from the environment is prohibited when said barrier element is coupled to said syringe outlet portion.

2. The medical container according to claim 1, and wherein a luer portion is formed at the syringe outlet portion.

3. The medical container according to claim 2, and wherein a needle hub is coupled or integrally attached with said luer portion and said needle being fixedly attached to said needle hub.

4. The medical container according to claim 1, and wherein said barrier element is also coupled to said needle cover.

5. The medical container according to claim 2, and wherein said barrier element is coupled both to said luer portion and to said needle cover and is formed as a resilient sleeve.

6. The medical container according to claim 2, wherein said barrier element is removably attached to said luer portion.

7. The medical container according to claim 2, wherein said barrier element is fixedly attached to said luer portion.

8. The medical container according to claim 3, and wherein said barrier element is fixedly attached to or integrally molded with the needle hub.

9. The medical container according to claim 2, and wherein said barrier element is mounted over said needle cover and engages an outer portion of the luer portion.

10. The medical container according to claim 1, further comprising a vent element selectably operatively engaged with said barrier element to allow passage of fluid into an inner volume of said barrier element in a first operative orientation and prevent passage of fluid into the inner volume of the barrier element in a second operative orientation.

11. The medical container according to claim 10, whereas said vent element is operable by a user.

12. The medical container according to claim 10, whereas said vent element is operable upon exertion of pressure change.

13. The medical container according to claim 10, and whereas said vent element is fixedly attached to a package of said medical container.

14. The medical container with a protected needle according to claim 1, and further comprising a reinforcing sleeve received by said barrier element.

15. The medical container according to claim 1, and wherein the barrier element being mounted onto at least one of the syringe outlet and the needle cover in a pressure-fit manner.
